# EUROPEAN PATENT APPLICATION

(11) **EP 4 813 459 A1**
(43) Date of publication of application: **30.09.2026**
(21) Application number: 25305418.3
(22) Date of filing: 24.03.2025
(51) Int. Cl.: A61P 31/12, A61P 35/00, C07K 4/02, C07K 16/00, C07K 16/08, G01N 33/53, A61K 39/00, A61K 39/12

(54) **ONCOGENIC HCMV-IE1 PROTEIN AND ITS USE IN THE PREVENTION AND TREATMENT OF CANCERS**

(71) Applicant: Apex Biosolutions, 25220 Roche-Lez-Beaupré (FR)
(72) Inventor: HERBEIN, Georges, 25000 BESANCON (FR)
(74) Representative: Ipsilon

(57) **Abstract**

The present invention relates to an agent that binds partially or completely to an amino acid sequence of formula (I) of the IE1 protein from HCMV:

LX₁X₂X₃E (I)

wherein L is a leucine;
X₁, X₂ and X₃ are, independently, any naturally occurring amino acid; and E is a glutamic acid.

It further relates to a pharmaceutical composition, uses and a diagnostic kit, as well as methods for evaluating the oncogenic property of an HCMV strain and for determining the risk of carcinogenesis in an individual or for evaluating a cancer prognosis in an individual.

## Description

### FIELD OF THE INVENTION

The invention relates to agents that are able to bind to the IE1 protein from HCMV and their use as therapeutic agents, as well as the use of the IE1 protein and its nucleotide sequence from HCMV as a diagnostic tool or a therapeutic target, with respect to cancer.

### BACKGROUND OF THE INVENTION

The term "cancer" refers to a broad group of different diseases, all involving unregulated cell growth. In cancer, the cells of a tissue divide and grow uncontrollably, leading to the formation of malignant tumors, and eventually to the invasion of neighboring or distant tissues or organs via the lymphatic system or blood circulation, and the formation of metastases.

Glioblastoma is an aggressive and common type of brain tumor that arises from glial cells, specifically astrocytes. It is known for its rapid growth and resistance to many treatment strategies. Glioblastoma tumors are characterized by a high degree of heterogeneity, both in terms of their molecular profiles and their response to treatment. The prognosis for glioblastoma is generally poor, with a median survival of around 15 months, even with treatment involving surgery, radiation therapy, and chemotherapy. Currently, there is no efficient treatment available to cure glioblastoma.

Breast cancer (or malignant breast tumor) is a type of cancer originating from breast tissues, most commonly from the inner lining of milk ducts or lobules that supply the ducts with milk. Although the vast majority of breast cancers in humans affect women, some men may also be affected. Worldwide, breast cancer accounts for about 25% of all cancers in women. Like most cancers, breast cancer includes a set of heterogeneous tumors with very different clinical characteristics, pathological progressions, and therapeutic responses. Thus, the intrinsic characteristics of the tumors, such as histological, immunopathological, or molecular traits, lead to the classification of breast tumors into different groups with often varying prognoses. Additionally, the extrinsic characteristics of the tumors, such as the microenvironment, can impact the prognosis of breast cancer (Bertos & Park, J Clin Invest, 2011, 121:3789-3796).

Ovarian cancer is a type of cancer that originates in the ovaries. It is often diagnosed at a late stage due to its subtle symptoms, which may include bloating, abdominal pain, and changes in appetite or urinary frequency. As a result, ovarian cancer is one of the most dangerous cancers for women, with a high mortality rate. The majority of ovarian cancers are epithelial in origin, meaning they begin in the thin layer of tissue covering the ovaries. However, ovarian cancer can also arise from other cell types, such as germ cells or stromal cells.

Prostate cancer is one of the most common types of cancer among men. It typically originates in the prostate gland and may present with symptoms such as difficulty urinating or pelvic discomfort. Like breast cancer, prostate cancer is often heterogeneous, with different molecular and genetic profiles influencing treatment responses and patient outcomes.

Prognosis and survival rates can vary widely depending on the cancer type, stage, and the treatment strategy adopted. Therefore, a better understanding of the underlying characteristics of carcinogenesis, cancer heterogeneity, as well as the mechanisms and etiology involved, is essential to improve treatment strategies, develop new therapies, and enhance the prognosis of patients.

Numerous factors are known to increase the risk of carcinogenesis, such as tobacco use, certain radiation, lack of physical activity, poor diet, obesity, environmental pollutants, and certain infections, particularly viral infections.

Oncogenic viruses, or viruses with oncogenic properties, are viruses that have the ability to induce different types of tumors in certain individuals, sometimes many years after the initial infection. Some viruses become oncogenic when they persist after infection in an episomal form, such as Epstein-Barr virus or Kaposi's sarcoma-associated herpesvirus. Others, like papillomaviruses, only become carcinogenic when they integrate into the host cell genome. The oncogenic properties of certain viruses involve direct mechanisms, such as the insertion of viral oncogenes into the host cell or the activation of existing host proto-oncogenes. Other viruses exhibit their oncogenicity by inducing chronic inflammation, as is the case with liver cancer induced by hepatitis C virus.

The main viruses associated with cancers in humans are human papillomavirus, hepatitis B and C viruses, Epstein-Barr virus, human T-lymphotropic virus (HTLV), Kaposi's sarcoma-associated herpesvirus (KSHV), and Merkel cell polyomavirus.

Human cytomegalovirus (HCMV) is a herpesvirus that infects between 40% and 95% of the population worldwide, usually without symptoms. The host immune response keeps the virus in a latent stage, although HCMV can reactivate in an inflammatory context, which could result in sequential lytic/latent viral cycles during the lifetime and thereby participate in HCMV genomic diversity in humans.

Recent studies have suggested that HCMV could play a role in the development and progression of several types of cancers, including breast cancer, ovarian cancer, prostate cancer, and glioblastoma. While the direct link between HCMV and these cancers is not yet fully understood, there is growing evidence indicating that HCMV infection could influence tumorigenesis through mechanisms such as inducing chronic inflammation, activating cellular pathways that promote tumor growth, downregulation of tumor suppressors such as p53 and retinoblastoma proteins and modulating the immune system to allow the tumor to escape immune surveillance.

In the case of breast cancer, several studies have detected the presence of HCMV in tumor tissues, suggesting that the virus may be involved in disease progression. Research has shown that HCMV could promote cell survival and resistance to apoptosis, two key features of breast tumors. Similarly, in ovarian cancer, HCMV infections have been observed in tumor cells, favoring cell proliferation and resistance to cell death, and it has been suggested that the virus may contribute to an immunosuppressive tumor environment, thereby promoting tumor growth and metastasis.

For prostate cancer, although studies are less extensive, preliminary research has revealed that HCMV may be present in certain tumor cells and could play a role in chronic inflammation, a well-known factor in prostate cancer progression. Furthermore, HCMV might interact with key molecular pathways involved in regulating cell growth and metastasis and directly favor transformation of prostate epithelial cells.

Regarding glioblastoma, one of the most aggressive brain cancers, several studies have shown that HCMV could be present in tumor cells, and recent research suggests that the virus may play a crucial role in tumor progression. It has been demonstrated that HCMV could interact with cellular pathways that promote tumor cell survival and resistance to treatment, such as resistance to apoptosis and activation of signaling pathways related to tumor growth. The tumor microenvironment, altered by viral infection, could also contribute to promoting invasion and metastasis, which are characteristic of glioblastoma. Persistent HCMV infection could thus support a state of chronic inflammation and immune dysfunction, contributing to the rapid evolution of the tumor.

Although the exact role of HCMV in these cancers is not yet definitively established, these observations suggest that the virus could be a co-factor in the oncogenic process, particularly in immunocompromised populations or those with persistent infection. These findings open the door to new therapeutic approaches, including the use of antiviral treatments specifically targeting HCMV, in conjunction with conventional treatments for these cancers.

The IE1 protein (Immediate Early 1) of HCMV is a crucial viral protein that plays an essential role in the early stages of HCMV infection. IE1 is part of the class of immediate-early proteins, meaning it is among the first proteins expressed during viral infection and is pivotal for initiating the viral replication cycle. Beyond its role in the viral life cycle, the IE1 protein has been implicated in the oncogenic potential of HCMV due to its ability to interact with host cell processes, influencing cellular behavior in ways that may contribute to tumorigenesis. IE1 protein is coded by the gene UL123. Its Genbank reference is ALN67102.1.

In view of the above, there is a need for agents that are able to bind to and inhibit HCMV, and in particular that are able to bind and inhibit the IE1 protein of HCMV.

In particular, there is a need for agents that can be used as biomarkers and diagnostic tools for determining the oncogenic property of an HCMV strain, for determining the risk of carcinogenesis or for evaluating a cancer prognosis in an individual.

There is also a need for agents that can act as therapeutic agents in the prevention and/or treatment of cancer, in particular in cancers that express the IE1 protein of HCMV.

Finally, there is a need for agents that can bind and inhibit therapeutic targets, in particular therapeutic targets in HCMV, in particular in the IE1 protein of HCMV, with respect to cancer.

The present invention aims to address one or more of the above-mentioned problems.

### SUMMARY OF THE INVENTION

The inventors have shown that a specific motif of the IE1 protein of HCMV was responsible for the oncogenic properties of the virus.

Indeed, the examples of the present text show that the transduction of cells with plasmids wherein the IE1 protein is mutated within this particular motif results in the loss of the oncogenic properties of the transduced cells.

As such, the motif of the IE1 protein of HCMV identified by the inventors can serve as a therapeutic target in the prevention and/or treatment of cancers, in particular of cancers that express the IE1 protein of HCMV.

The motif of the IE1 protein of HCMV identified by the inventors can also serve as a tool to determine whether an HCMV strain may be oncogenic as well as to determine the risk of carcinogenesis or to evaluate a cancer prognosis in an individual.

### BRIEF DESCRIPTION OF THE FIGURES

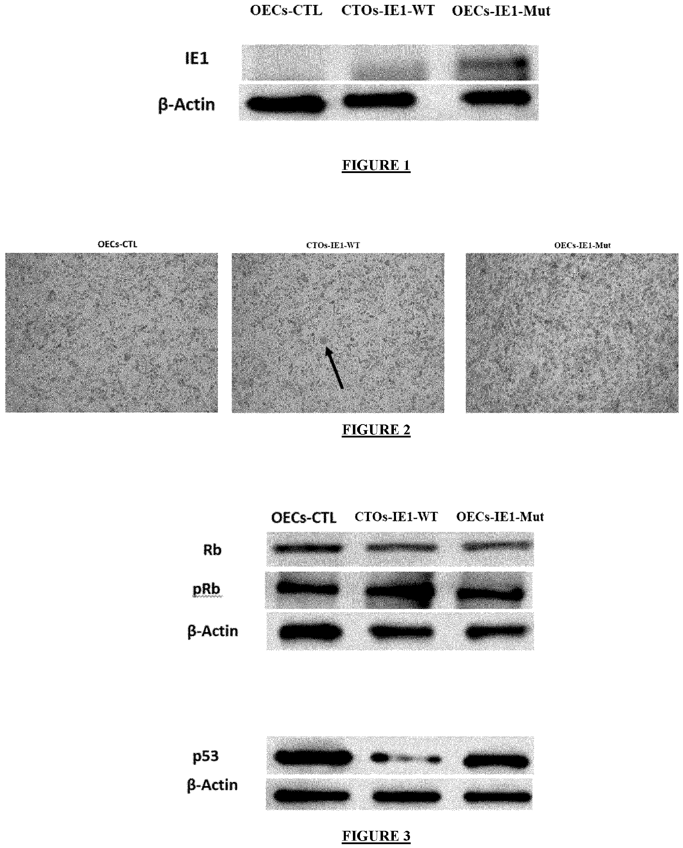

**Figure 1** shows the Western Blot analysis for IE1 in the different cell cultures, from left to right on the gel: OECs-CTL (cells transduced with pHCMV-GFP plasmid), CTOs-IE1-WT (cells transduced with pHCMV-DB-IE1-wt plasmid) and OECs-IE1-Mut (cells transduced with pHCMV-DB-IE1-Mut plasmid). β-actin was used as a loading control to ensure equal protein loading across the samples.
**Figure 2** shows microscopic images with a magnification of x20 captured for OECs transduced with GFP (image on the left) (OECs-CTL), IE1-DB-WT (image in the middle) (CTOs-IE1-WT) and IE1-DB-Mut plasmids (image on the right) (OECs-IE1-Mut) after 29 days of cell culture.
**Figure 3** shows the Western Blot analysis for Rb, pRb and p53 in the different cell cultures, from left to right on the gel: OECs-CTL (cells transduced with pHCMV-GFP plasmid), CTOs-IE1-WT (cells transduced with pHCMV-DB-IE1-wt plasmid) and OECs-IE1-Mut (cells transduced with pHCMV-DB-IE1-Mut plasmid). β-actin was used as a loading control to ensure equal protein loading across the samples.

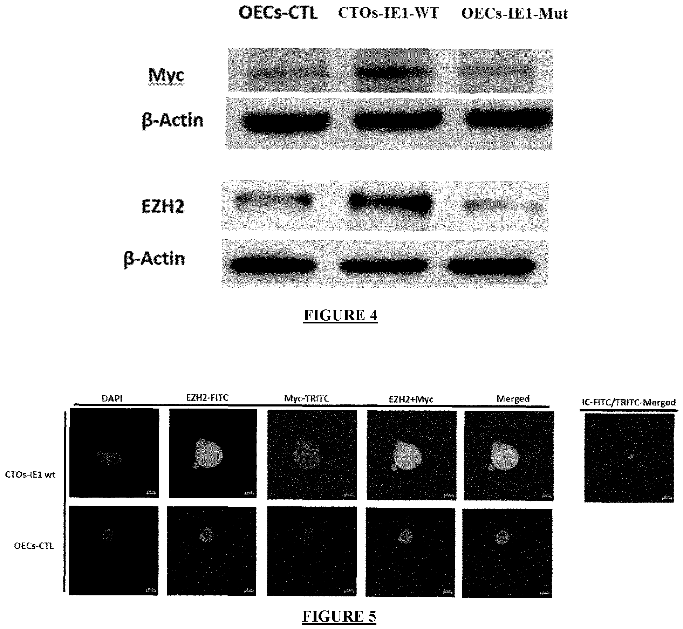

**Figure 4** shows the Western Blot analysis for Myc and EZH2 in the different cell cultures, from left to right on the gel: OECs-CTL (cells transduced with pHCMV-GFP plasmid), CTOs-IE1-WT (cells transduced with pHCMV-DB-IE1-wt plasmid) and OECs-IE1-Mut (cells transduced with pHCMV-DB-IE1-Mut plasmid). β-actin was used as a loading control to ensure equal protein loading across the samples.
**Figure 5** shows confocal imaging revealing Myc and EZH2 localization in OECs-CTL (cells transduced with pHCMV-GFP plasmid) and CTOs-IE1-WT (cells transduced with pHCMV-DB-IE1-wt plasmid) cells, with nuclei counterstained using DAPI. Images were captured at a magnification of ×63, with a scale bar of 10 µm.

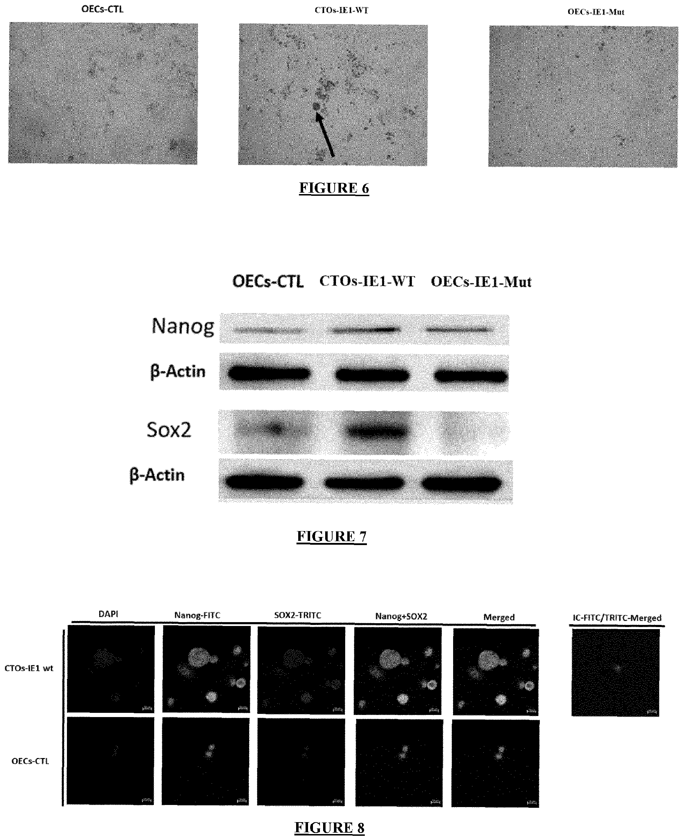

**Figure 6** shows microscopic images of colony formation assessed in soft agar seeded with OECs-CTL (cells transduced with pHCMV-GFP plasmid), CTOs-IE1-WT (cells transduced with pHCMV-DB-IE1-wt plasmid) and OECs-IE1-Mut (cells transduced with pHCMV-DB-IE1-Mut plasmid) cells. The resulting colonies were visualized under an inverted light microscope at a magnification of ×20.
**Figure 7** shows the Western Blot analysis for Nanog and Sox2 in the different cell cultures, from left to right on the gel: OECs-CTL (cells transduced with pHCMV-GFP plasmid), CTOs-IE1-WT (cells transduced with pHCMV-DB-IE1-wt plasmid) and OECs-IE1-Mut (cells transduced with pHCMV-DB-IE1-Mut plasmid). β-actin was used as a loading control to ensure equal protein loading across the samples.
**Figure 8** shows confocal microscopy revealing the expression of Nanog and SOX2 proteins in OECs-CTL (cells transduced with pHCMV-GFP plasmid) and CTOs-IE1-WT (cells transduced with pHCMV-DB-IE1-wt plasmid) cells, with nuclei counterstained using DAPI. Images were captured at a magnification of ×63, with a scale bar of 10 µm.

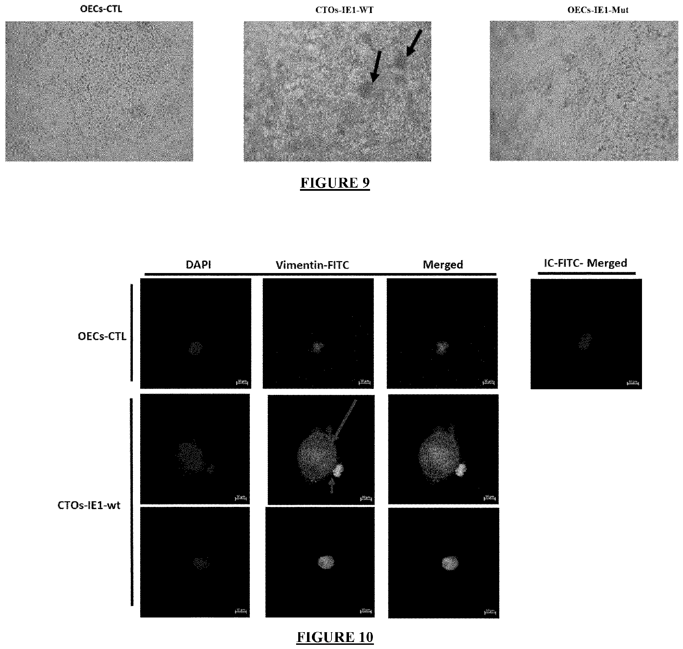

**Figure 9** shows microscopic images of the formation of spheroids in the presence of methylcellulose within the CTOs-IE1-WT (cells transduced with pHCMV-DB-IE1-wt plasmid) cultures, observed at a magnification of ×20.
**Figure 10** shows confocal microscopy revealing vimentin protein expression in OECs-CTL (cells transduced with pHCMV-GFP plasmid) and CTOs-IE1-WT (cells transduced with pHCMV-DB-IE1-wt plasmid) cells, with nuclei counterstained using DAPI. Images were captured at a magnification of ×63, with a scale bar of 10 µm.

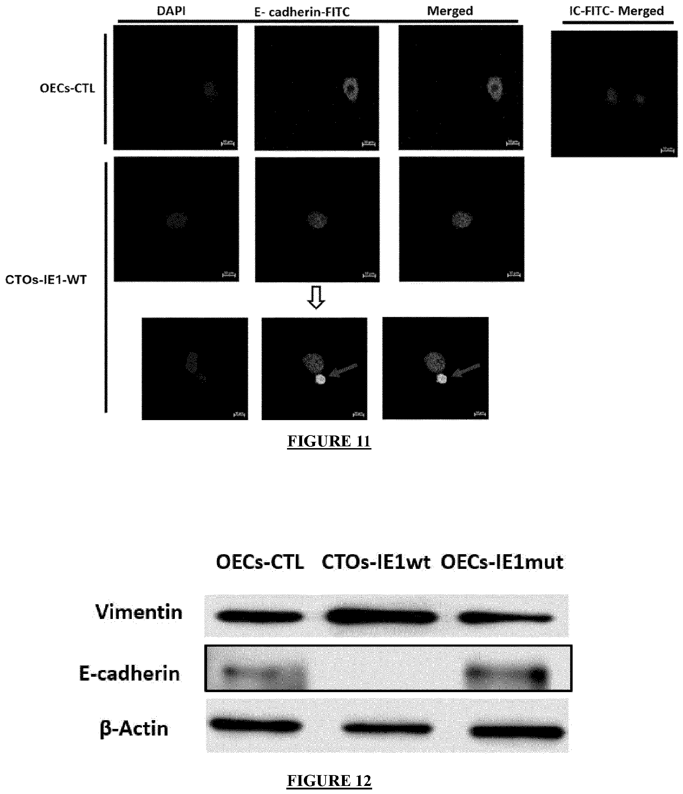

**Figure 11** shows confocal microscopy revealed E-cadherin protein expression in OECs-CTL (cells transduced with pHCMV-GFP plasmid) and CTOs-IE1-WT (cells transduced with pHCMV-DB-IE1-wt plasmid) cells, with nuclei counterstained using DAPI. Images were taken at a magnification of ×63, with a scale bar of 10 µm.
**Figure 12** shows the Western Blot analysis for vimentin and E-cadherin in the different cell cultures, from left to right on the gel: OECs-CTL (cells transduced with pHCMV-GFP plasmid), CTOs-IE1-WT (cells transduced with pHCMV-DB-IE1-wt plasmid) and OECs-IE1-Mut (cells transduced with pHCMV-DB-IE1-Mut plasmid). β-actin was used as a loading control to ensure equal protein loading across the samples.

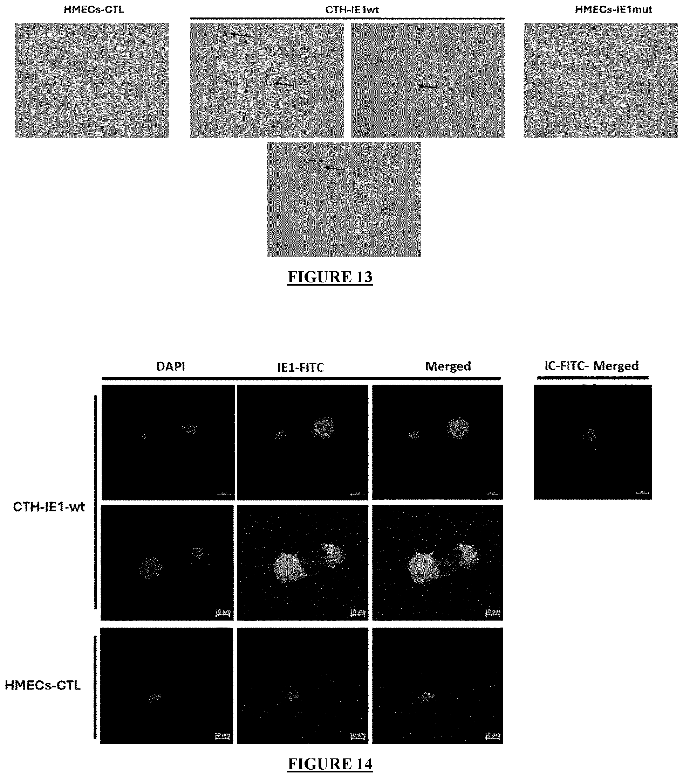

**Figure 13** shows microscopic images of HMECs transduced with GFP (HMECs-CTL), IE1-DB-WT (CTH-1E1wt) and IE1-DB-Mut (HMECS-1E1mut) plasmids. In the CTH-IE1-WT cultures, black arrows indicate the presence of large cells and cells filled with lipid droplets. These observations were made at a magnification of ×40.
**Figure 14** shows confocal microscopy images of HMECs-CTL and CTH-IE1-wt cells showing the localization of IE1, with nuclei counterstained using DAPI. The images were captured at a magnification of ×63, with a scale bar of 10 µm.

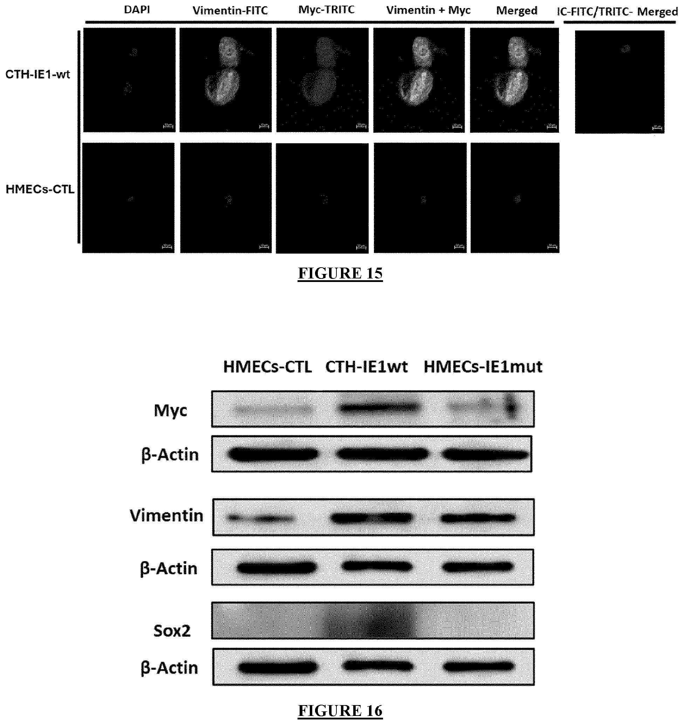

**Figure 15** shows confocal imaging revealing the expression of vimentin and Myc in HMECs-CTL and CTH-IE1-wt cells, with nuclei counterstained using DAPI. The images were captured at a magnification of ×63, with a scale bar of 10 µm,
**Figure 16** shows the Western Blot analysis for Myc, vimentin and E-cadherin in the different cell cultures, from left to right on the gel: HMECs-CTL, CTH-IE1wt, and HMECs-IE1mut cells. β-actin was used as a loading control to ensure equal protein loading across the samples.

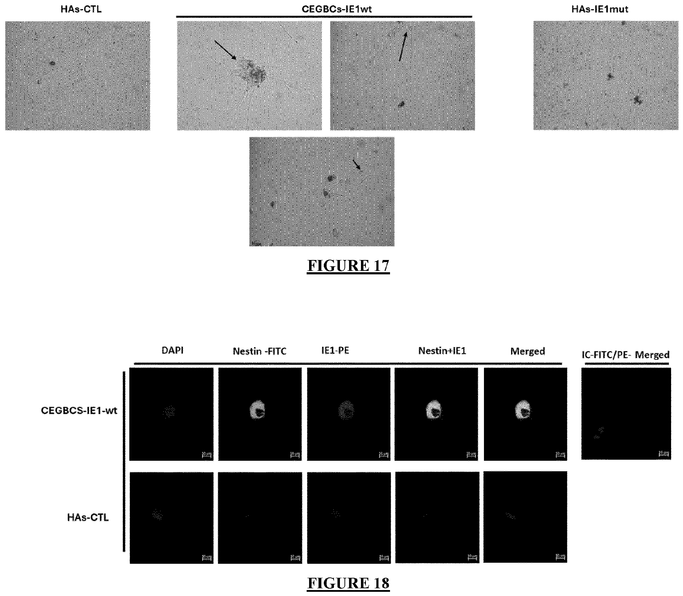

**Figure 17** shows microscopic images taken of HAs transduced with GFP (HAs-CTL), IE1-DB-WT (CEGBCs-IE1-wt), and IE1-DB-Mut (HAs-IE1mut) plasmids. These images were captured at a magnification of ×40.
**Figure 18** shows confocal microscopy images of HAs-CTL and CEGBCS-IE1-wt cells showing the presence of IE1 and nestin, with nuclei counterstained using DAPI. The images were captured at a magnification of ×63, with a scale bar of 10 µm.

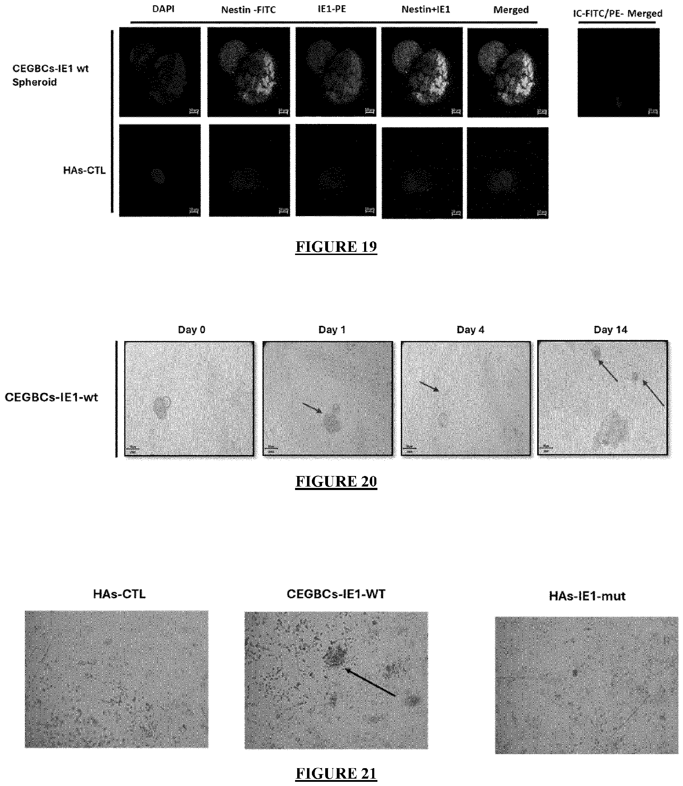

**Figure 19** shows confocal imaging revealing the expression of nestin and IE1 in HAs-CTL and CEGBCS-IE1-wt cells, with nuclei counterstained using DAPI. The images were captured at a magnification of ×63, with a scale bar of 10 µm.
**Figure 20** shows microscopy images of the 3D invasion assay conducted over 14 days showing CEGBCS-IE1-wt spheroids embedded in type-1 collagen in the presence of HCl. Arrows highlight the invasive behavior of the cells as they migrated out of the spheroids. These images were captured at a magnification of ×200, with a scale bar of 50 µm.
**Figure 21** shows microscopic images capturing the generation of spheroids in the presence of methylcellulose in CEGBCs-IE1-wt cells. These images were taken at a magnification of ×20.

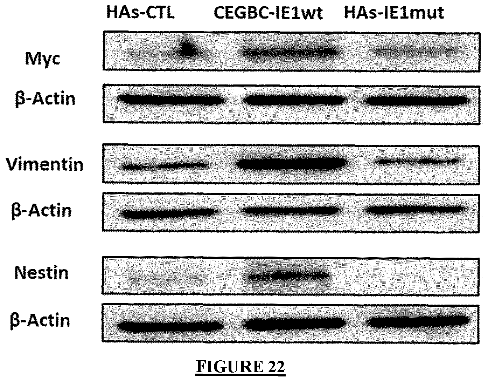

**Figure 22** shows the Western Blot analysis for Myc, vimentin and nestin in the different cell cultures, from left to right on the gel: HAs-CTL, CEGBCs-IE1wt, and HAs-IE1mut cells. β-actin was used as a loading control to ensure equal protein loading across the samples.

### DETAILED DESCRIPTION OF THE INVENTION

In the context of the present invention, the terms *"treat"* or *"treatment",* in particular when used in relation with a disease, denotes slowing down or stopping the development or progression of the disease, causing regression of the associated clinical symptoms or relieving the disease partially or completely. The term "treat" must also be understood as encompassing the reduction of number of relapses and/or the reduction of intensity of the relapses and/or the increase of the time separating two relapses of a disease according to the present invention, in particular of a cancer, more particularly a cancer that expresses the IE1 protein from HCMV, more particular a cancer selected from the group consisting of glioblastoma, breast cancer, ovarian cancer and prostate cancer.

In the context of the present invention, the term *"prevent"* or *"prevention"* denotes the reduction to a lesser degree of the risk or of the probability of occurrence of a given phenomenon, that is to say, in the present invention, a cancer. The term *"prevent"* must also be understood as encompassing the reduction to a lesser degree of the risk or of the probability of relapse of a disease according to the present invention, in particular of a chronic one.

A *"wild type"* or "*WT*" amino acid sequence refers to the original, unaltered sequence of amino acids found in a naturally occurring gene or protein in a given species. A *"mutated"* amino acid sequence refers to a sequence in which one or more amino acids have been altered due to genetic mutations, potentially resulting in changes to the protein's structure or function. For example, the wild type amino acid sequence of IE1 protein of HCMV-DB is the amino acid sequence set forth as SEQ ID NO: 25. A mutated version of this amino acid sequence is an amino acid sequence wherein one or more amino acids have been altered.

As used herein, the term *"cancer"* refers to any member of a class of diseases or disorders characterized by uncontrolled division of cells and the ability of these cells to invade other tissues, either by direct growth into adjacent tissue through invasion or by implantation into distant sites by metastasis. Metastasis is defined as the stage in which cancer cells are transported through the bloodstream or lymphatic system. The term cancer according to the present invention also comprises cancer metastases and relapse of cancer. Examples of cancers more particularly considered in the present invention are provided further. Cancers may in particular refer herein to a cancer selected from the group consisting of myeloma; melanoma; breast cancer, in particular triple negative breast cancer; prostate cancer; primary and metastatic colorectal cancer, in particular colon cancer or metastatic colon cancer; glioblastoma; lung cancer, in particular non small cell lung cancer (NSCLC) or small cell lung cancer (SCLC); liver cancer, in particular hepatocarcinoma or cholangiocarcinoma; primary and metastatic pancreas cancer, in particular pancreatic adenocarcinoma; lymphoma, in particular B-cell or T-cell lymphoma, more particularly T-cell lymphoma; gastric cancer; head and neck cancer; ovarian cancer; sarcoma; acute and chronic leukemia; osteosarcoma; anal cancer; testicular cancer; uterus cancer; thyroid cancer; cancer of the central nervous system; gastrointestinal stromal cancer; epidermal carcinoma and oesophageal cancer.

As used herein, the terms *"cancer that expresses the IE1 protein from HCMV"* refers to a tumor where HCMV is active and producing the IE1 protein. In a particular embodiment, the cancer that expresses the IE1 protein from HCMV is selected from the group consisting of glioblastoma, breast cancer, ovarian cancer and prostate cancer, more particularly the cancer is glioblastoma.

As defined in the present specification, an *"individual in need thereof"* is in particular an individual that suffers from cancer, in particular an individual that suffers from cancer that expresses the IE1 protein from HCMV, more particularly is an individual that suffers from glioblastoma, breast cancer, ovarian cancer and/or prostate cancer.

*"Pharmaceutically"* or *"pharmaceutically acceptable"* refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

As used herein, *"pharmaceutically acceptable carriers"* includes any and all solvents, dispersion media, coatings, antibacterial and antifungal agents, and the like that are physiologically compatible. Examples of suitable carriers, diluents and/or excipients include one or more of water, amino acids, saline, phosphate buffered saline, buffer phosphate, acetate, citrate, succinate; amino acids and derivates such as histidine, arginine, glycine, proline, glycylglycine; inorganic salts NaCl, calcium chloride; sugars or polyalcohols such as dextrose, glycerol, ethanol, sucrose, trehalose, mannitol; surfactants such as Polysorbate 80, polysorbate 20, poloxamer 188; and the like, as well as combination thereof. In many cases, it will be preferable to include isotonic agents, such as sugars, polyalcohols, or sodium chloride in the composition, and formulation may also contain an antioxidant such as tryptamine and a stabilizing agent such as Tween 20. Suitable excipients, as well as pharmaceutical formulation requirements, are described in "Remington: The Science & Practice of Pharmacy", which is a reference work in the field.

The terms *"sequence homology"* or *"sequence identity"* or *"homology"* or *"identity"* are used interchangeably herein. For the purpose of the invention, it is defined here that in order to determine the percentage of sequence homology or sequence identity of two amino acid sequences or of two nucleic acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region. A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences or between two nucleotide sequences may be determined using the Needleman and Wunsch algorithm for the alignment of two sequences. (Needleman, S. B. and Wunsch, C. D. (1970) J. Mol. Biol. 48, 443-453). Both amino acid sequences and nucleotide sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. And Bleasby,A. Trends in Genetics 16, (6) pp276-277, http://emboss.bioinformatics.nl/). For protein sequences EBLOSUM62 is used for the substitution matrix. For nucleotide sequence, EDNAFULL is used. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid or identical nucleotide in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of nucleotide and amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

It is to be noted that the term "a" or "an" entity refers to one or more of that entity; for example, "an antibody," is understood to represent one or more antibodies. As such, the terms "a" (or "an"), "one or more" and "at least one" can be used interchangeably herein.

Throughout this specification and embodiments, the words "have" and "comprise," or variations such as "has", "having", "comprises" or "comprising" will be understood to imply the inclusion of a stated integer or group of integers but not the exclusion of any other integer or group of integers. The words "have" and "comprise" or variations such as "has", "having", "comprises" or "comprising" will be understood to imply the inclusion of the stated element(s) (such as a composition of matter or a method step) but not the exclusion of any other elements. The term "consisting of" implies the inclusion of the stated element(s), to the exclusion of any additional elements. The term "consisting essentially of" implies the inclusion of the stated elements, and possibly other element(s) where the other element(s) do not materially affect the basic characteristic(s) of the disclosure. It is understood that the different embodiments of the disclosure using the term "comprising" or equivalent cover the embodiments where this term is replaced with "comprising only", "consisting of" or "consisting essentially of".

It is understood that wherever aspects are described herein with the language "comprising" otherwise analogous aspects described in terms of "consisting of" and/or "consisting essentially of" are also provided.

Furthermore, "and/or" where used herein is to be taken as specific disclosure of each of the two specified features or components with or without the other. Thus, the term "and/or" as used in a phrase such as "A and/or B" herein is intended to include "A and B", "A or B", "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

### Agents able to inhibit the expression or activity of IE1 protein from HCMV

The present invention relates to an agent that binds partially or completely to an amino acid sequence of formula (I) of the IE1 protein from HCMV.

By *"an agent that binds partially or completely"* to a given amino acid sequence, it is understood an agent that, when it binds to the amino acid sequence, is able to inhibit the activity of the IE1 protein from HCMV.

IE1 protein, or immediate-early-1 protein, is one of the first proteins expressed after the HCMV virus infects a cell, playing a key role in viral replication and modulating the host cell environment to facilitate viral replication.

By an agent that *"binds partially"* to a given amino acid sequence, it is understood an agent that interacts with a specific region of the amino acid sequence, forming a covalent or non-covalent, often transient, association without fully occupying or binding to the entire sequence.

On the contrary, by an agent that *"binds completely"* to a given amino acid sequence, it is understood an agent that forms a covalent or non-covalent interaction with the entire amino acid sequence, typically resulting in a complete and strong binding affinity between the agent and the amino acid sequence.

The agent according to the invention binds partially or completely to an amino acid sequence of formula (I) of the IE1 protein from HCMV:

LX₁X₂X₃E (I)

wherein L is a leucine;
X₁, X₂ and X₃ are, independently, any naturally occurring amino acid, in particular X₂ is a threonine; and
E is a glutamic acid.

In particular, the amino acid sequence of formula (I) has the amino acid sequence set forth as SEQ ID NO: 20.

By *"naturally occurring amino acid"*, it is understood an amino acid that is found in nature and is commonly integrated into proteins in living organisms. Naturally occurring amino acids consist of the following group of amino acids: Alanine (A), Arginine (R), Asparagine (N), Aspartic acid (D), Cysteine (C), Glutamine (Q), Glutamic acid (E), Glycine (G), Histidine (H), Isoleucine (I), Leucine (L), Lysine (K), Methionine (M), Phenylalanine (F), Proline (P), Serine (S), Threonine (T), Tryptophan (W), Tyrosine (Y), and Valine (V).

According to a particular embodiment, the agent binds partially or completely to an amino acid sequence of formula (II) of the IE1 protein:

X₄X₅X₆LX₁X₂X₃EX₇X₈ (II)

wherein E is a glutamic acid;
L is a leucine;
X₁, X₂, X₃, X₆ and X₇ are, independently, any naturally occurring amino acid, in particular X₂ is a threonine;
X₈ is a naturally occurring hydrophobic amino acid, in particular is a valine or a leucine, more particularly is a valine; and

X₄ and X₅ are, independently, a naturally occurring acidic amino acid, in particular X₄ and X₅ are a glutamic acid. In particular, the amino acid sequence of formula (II) has the amino acid sequence set forth as SEQ ID NO: 21.

By *"a naturally occurring acidic amino acid"*, it is understood an amino acid whose side chains can carry a negative charge et certain pH values. Such naturally occurring acidic acids consist of Glutamic acid (E) and Aspartic acid (D).

By *"a naturally occurring hydrophobic amino acid"*, it is understood an amino acid with a nonpolar side chain. Such naturally occurring hydrophobic amino acids consist of the following amino acids: Alanine (A), Valine (V), Leucine (L), Isoleucine (I), Phenylalanine (F), Tryptophan (W), Methionine (M), and Proline (P).

According to a particular embodiment, X₁, X₂, X₃ and, if present, X₆ and X₇, are, independently, selected from the group consisting of a serine, a lysine, a threonine, a phenylalanine and a glutamine.

In a particular embodiment, X₁ is a lysine; and/or X₂ is a threonine; and/or X₃ is a phenylalanine; and/or, if present, X₆ is a serine; and/or, if present, X₇ is a glutamine.

More particularly, X₁ is a lysine, X₂ is a threonine, X₃ is a phenylalanine, and, if present, X₆ is a serine and, if present, X₇ is a glutamine.

In a particular embodiment, X₁ is a lysine; and/or X₂ is a threonine; and/or X₃ is a phenylalanine; and/or, if present, X₄ and X₅ are a glutamic acid; and/or, if present, X₆ is a serine; and/or, if present, X₇ is a glutamine; and/or if present, X₈ is a valine.

More particularly, X₁ is a lysine, X₂ is a threonine, X₃ is a phenylalanine, and, if present, X₄ and X₅ are a glutamic acid; and, if present, X₆ is a serine and, if present, X₇ is a glutamine; and, if present, X₈ is a valine. According to a particular embodiment, the amino acid sequence of formula (I) is the amino acid sequence LKTFE set forth as SEQ ID NO: 22.

According to a particular embodiment, the amino acid sequence of formula (II) is the amino acid sequence EESLKTFEQV set forth as SEQ ID NO: 23.

According to a particular embodiment, the amino acid sequence is the sequence set forth as SEQ ID NO: 24 (EESLKTFEQVTEDCNENPELDVL).

An agent that binds partially or completely to the amino acid sequence as defined above may be selected from the group consisting of small inhibiting molecules, peptides, peptidomimetics, antibodies, antibody fragments, aptamers, macrocycles, molecular glues, synthetic derivatives of natural ligands, and nanobodies.

In a particular embodiment, the agent that binds partially or completely to the amino acid sequence as defined above may be a small inhibiting molecule.

*"Small inhibiting molecules"* as defined herein as compounds that can bind to specific enzymes or receptors, thereby blocking their activity and modulating biological processes.

According to a particular embodiment, the agent that binds partially or completely to the amino acid sequence as defined above may be an antibody or an antibody fragment.

The term *"antibody"* as used herein refers to immunoglobulin molecules and immunologically active portions of immunoglobulin molecules, i.e., molecules that contain an antigen binding site that immunochemically binds an antigen. As such, the term antibody encompasses not only whole antibody molecules, but also variants (including derivatives) of antibodies. In natural antibodies, two heavy chains are linked to each other by disulfide bonds and each heavy chain is linked to a light chain by a disulfide bond. There are two types of light chain, lambda (l) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from non-hypervariable or framework regions (FR) influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated L-CDR1, L-CDR2, L-CDR3 and

H-CDR1, H-CDR2, H-CDR3, respectively. An antigen-binding site, therefore, includes six CDRs, comprising the CDR set from each of a heavy and a light chain V region. Framework Regions (FRs) refer to amino acid sequences interposed between CDRs. The well-known Kabat numbering system is used in the present text for defining antibodies according to the invention, and in particular to define their CDRs.

In the present text, the term *"antibody"* is used in the broadest sense and includes fully assembled antibodies, monoclonal antibodies, polyclonal antibodies, multi-specific antibodies (e.g., bispecific antibodies), antibody fragments and recombinant peptides comprising the forgoing as long as they exhibit the desired biological activity defined herein, i.e. that of binding partially or completely to the amino acid sequence of the invention as defined above.

The term *"chimeric antibody"* refers to an antibody which comprises a VH domain and a VL domain of an antibody which are derived from one species and the constant domain which is derived from another species, for example an antibody in which the variable region sequences are derived from a mouse antibody and the constant region sequences are derived from a human antibody.

According to the invention, the term *"humanized antibody"* refers to an antibody having variable region framework and constant regions from a human antibody but which retains the mouse sequence origin of the CDRs of the variable V regions.

The terms *"monoclonal antibody"* or "monoclonal antibody composition" as used herein refer to a preparation of antibody molecules of single molecular composition. A monoclonal antibody composition displays a single binding specificity and affinity for a particular epitope.

According to a particular embodiment, the agent that binds partially or completely to the amino acid sequence as defined above is an antibody fragment comprising a variable domain of a heavy chain and a variable domain of a light chain. In particular, the antibody fragment may be selected from the group consisting of Fv, Fab, F(ab')2, Fab', dsFv, scFv, sc(Fv)2 and diabodies.

A *"fragment of an antibody"* herein refers to a fragment of an intact antibody that retain the ability to specifically binds to a given antigen/ligand. Examples of fragment of an antibody include a Fab fragment, a monovalent fragment consisting of the VL, VH, CL and CH1 domains; a Fab' fragment, a monovalent fragment consisting of the VL, VH, CL, CH1 domains and hinge region; a F(ab')2 fragment, a bivalent fragment comprising two Fab' fragments linked by a disulfide bridge at the hinge region; an Fd fragment consisting of VH-CH1 domains of a single arm of an antibody, in particular of a heavy chain of an antibody; a single domain antibody (sdAb) fragment (Ward et al., 1989 Nature 341:544-546), which consists of a VH domain or a VL domain; and an isolated complementary determining region (CDR). Furthermore, although the two domains of the Fv fragment, VL and VH, are coded for by separate genes, they can be joined, using recombinant methods, by an artificial peptide linker that enables them to be made as a single protein chain in which the VL and VH regions pair to form monovalent molecules (known as single chain Fv (scFv); see, e.g., Bird et al., 1989 Science 242:423-426; and Huston et al., 1988 proc. Natl. Acad. Sci. 85:5879-5883). "dsFv" is a VH::VL heterodimer stabilized by a disulfide bond.

Divalent and multivalent antibody fragments can form either spontaneously by association of monovalent scFvs or can be generated by coupling monovalent scFvs by a peptide linker, such as divalent sc(Fv)2. Such single chain antibodies include one or more antigen-binding portions or fragments of an antibody. These antibody fragments are obtained using conventional techniques known to those skilled in the art, and the fragments are screened for utility in the same manner as are intact antibodies.

A unibody is another type of antibody fragment lacking the hinge region of IgG4 antibodies. The deletion of the hinge region results in a molecule that is essentially half the size of traditional IgG4 antibodies and has a univalent binding region rather than the bivalent binding region of IgG4 antibodies. Further details on UniBodies may be obtained by reference to WO 2007/059782, which is incorporated by reference in its entirety.

Fragments of antibodies can be incorporated into single domain antibodies, SMIP, maxibodies, minibodies, intrabodies, diabodies, triabodies and tetrabodies (see, e.g., Hollinger and Hudson, 2005, Nature Biotechnology, 23, 9, 1126-1136). The term "diabodies" "tribodies" or "tetrabodies" refers to small antibody fragments with multivalent antigen-binding sites (2, 3 or four), which fragments comprise a heavy-chain variable domain (VH) connected to a lightchain variable domain (VL) in the same polypeptide chain (VH-VL). By using a linker that is too short to allow pairing between the two domains on the same chain, the domains are forced to pair with the complementary domains of another chain and create two antigen-binding sites. Further details on domain antibodies and methods of their production are found in US 6,291,158; 6,582,915; 6,593,081; 6,172,197; and 6,696,245; US 2004/0110941; EP 1433846, 0368684 and 0616640; WO 2005/035572, 2004/101790, 2004/081026, 2004/058821, 2004/003019 and 2003/002609, each of which is herein incorporated by reference in its entirety.

Antigen binding fragments can be incorporated into single chain molecules comprising a pair of tandem Fd segments (VH-CH1-VH-CH1) which, together with complementary light chain polypeptides, form a pair of antigen binding regions (Zapata et al., 1995 Protein Eng. 8(10); 1057-1062 and U.S. Pat. No. 5,641,870).

Fab fragments can be obtained by treating an antibody with a protease, papain. Also, the Fab can be produced by inserting DNA encoding Fab of the antibody into a vector for prokaryotic expression system, or for eukaryotic expression system, and introducing the vector into a procaryote or eucaryote (as appropriate) to express the Fab.

F(ab')2 can be obtained by treating an antibody with a protease, pepsin. Also, the F(ab')2 can be produced by binding Fab' described below via a thioether bond or a disulphide bond.

Fab' can be obtained by treating F(ab')2 with a reducing agent, dithiothreitol. Also, the Fab' can be produced by inserting DNA encoding Fab' fragment of the antibody into an expression vector for prokaryote, or an expression vector for eukaryote, and introducing the vector into a prokaryote or eukaryote (as appropriate) to perform its expression.

An antigen-binding fragment may be variable heavy chain of a single domain antibody (VHH). Some VHHs may also be known as Nanobodies. Camelid single domain antibody (sdAb) is one of the smallest known antigen-binding antibody fragments (see, e.g., Hassanzadeh- Ghassabeh et al., Nanomedicine (Lond), 8:1013-26 (2013)). A basic VHH has the following structure from the N-terminus to the C-terminus: FR1-CDR1-FR2-CDR2-FR3-CDR3-FR4, in which FR1 to FR4 refer to framework regions 1 to 4, respectively, and in which CDR1 to CDR3 refer to the complementarity determining regions (CDR) 1 to 3.

In the context of the present invention, the term *"peptides"* refers to short chains of amino acids linked by peptide bonds.

For the purposes of this invention, *"peptidomimetics"* refer to molecules that mimic the structure and function of peptides.

Within this invention, *"aptamers"* refer to short, single-stranded DNA or RNA molecules that can bind to specific targets.

In this context, *"macrocycles"* refer to large, ring-shaped molecules that can bind to specific targets.

Herein, *"molecular glues"* refers to small molecules that facilitate the interaction between two proteins.

In the scope of this invention, *"synthetic derivatives of natural ligands"* refer to chemically modified versions of naturally occurring molecules that bind to specific targets.

Regarding this invention, "nanobodies" refers to small antibody fragments derived from camelid antibodies.

### Pharmaceutical composition

According to another object, the present invention relates to a pharmaceutical composition comprising an agent according to the invention and a pharmaceutically acceptable carrier.

The agent used in a pharmaceutical composition according to the invention may be any agent that binds partially or completely to an amino acid sequence of formula (I) of the IE1 protein from HCMV, as described throughout the present specification.

Pharmaceutically acceptable carriers are defined further above.

The pharmaceutical composition may be formulated for administration by a number of routes, including but not limited to oral, intravenous, intramuscular, subcutaneous, transdermal, inhalation, rectal, sublingual, intranasal, and ocular. According to a particular embodiment, the pharmaceutical composition is formulated for intramuscular or subcutaneous administration.

According to a particular embodiment, the pharmaceutical composition is a vaccine.

In a particular embodiment, the pharmaceutical composition further comprises at least one anticancer drug different from the agent according to the invention, and/or comprises at least one chemotherapy treatment, and/or comprises at least one anti-CMV treatment.

In some embodiments, the pharmaceutical composition further comprises at least one anticancer drug different from the agent according to the invention.

The at least one anticancer drug may be selected from chemotherapy agents, such as cisplatin, paclitaxel, doxorubicin, methotrexate, vincristine, carboplatin, temozolomide or oxaplatin; targeted therapy agents such as imatinib, erlotinib, sunitinib, rituximab, bevacizumab or lenalidomide; immunotherapy agents such as pembrolizumab or nivolumab: hormonal therapy agents such as tamoxifen; and monoclonal antibodies such as Herceptin, rituximab or bevacizumab.

According to a particular embodiment, the at least one anticancer drug is selected from the group consisting of monoclonal antibodies, in particular selected from the group consisting of anti-CD19, anti-CD20, anti-CD30, anti-CD137, anti-CTLA4, anti-TIM-3, anti-B7-H3, anti-CD123, anti-CD134, anti-CD154, anti-LAG-3, anti-CD227, anti-BTNA3, anti-CD39, anti-CD73, anti-CD115, anti-CD47, anti-SIRP alpha, anti-SIRP gamma, anti-CD28, anti-NCR, anti-NKp46, anti-NKp30, anti-NKp44, anti-NKG2D, anti-PD1, anti-PDL1, mogamulizumab, obinutuzumab, polatuzumab vedotin, Yttrium Y 90-ibritumomab tiuxetan, mosunetuzumab, cetuximab, atezolizumab/bevacizumab, anti-VEGF antibodies, anti-DNAM-1 monoclonal antibodies and mixtures thereof.

According to a particular embodiment, the pharmaceutical composition further comprises at least one chemotherapy treatment, in particular a chemotherapy treatment selected from the group consisting of a chemotherapy regimen consisting of Cyclophosphamide, Hydroxydaunorubicin, Oncovin and Prednisone (CHOP); a chemotherapy regimen consisting of Cyclophosphamide, Hydroxydaunorubicin, Oncovin, Etoposide and Prednisone (CHOEP); HDAC inhibitors; ibrutinib; acalabrutinib; zanubrutinib; copanlisib; venetoclax; folfirinox; cisplatin; doxorubicin; irinotecan; liposomal irinotecan; oxaliplatin; Nap paclitaxel; paclitaxel; gemcitabine; sorafenib; lenvatinib; imatinib; sunitinib; regorafenib; mTOR inhibitors and mixtures thereof.

According to a particular embodiment, the pharmaceutical composition further comprises at least one anti-CMV treatment, in particular comprises at least one anti-CMV treatment selected from the group consisting of ganciclovir, valganciclovir, foscarnet, cidofovir, letermovir, maribavir, aciclovir, and immune globulin (IVIG). An anti-CMV treatment may comprise more than one compounds selected from the group above.

### Uses and medical methods of the invention

According to another aspect, the present invention provides an agent according to the invention or a pharmaceutical composition according to the present invention for their use in the prevention and/or treatment of cancer.

The agent used in the prevention and/or treatment of cancer according to the invention may be any agent that binds partially or completely to an amino acid sequence of formula (I) of the IE1 protein from HCMV, as described throughout the present specification.

The pharmaceutical composition used in the prevention and/or treatment of cancer may be any pharmaceutical composition as described throughout the present specification.

The present specification further provides a method for preventing and or treating cancer in an individual in need thereof, comprising at least the step of administering to said individual an agent according to the invention or a pharmaceutical composition according to the invention.

The present specification further provides the use of an agent according to the invention to manufacture a medicament for preventing and/or treating cancer.

The present specification further provides a pharmaceutical composition for the prevention and/or treatment of cancer comprising an agent according to the invention.

The present specification also provides a pharmaceutical composition for use in the prevention and/or treatment of cancer, comprising an agent according to the invention.

According to a particular embodiment, the cancer is a cancer that expresses the IE1 protein of HCMV, in particular the cancer is selected from the group consisting of glioblastoma, breast cancer, ovarian cancer and prostate cancer, more particularly the cancer is glioblastoma.

### Diagnostic or prognostic uses and kits

The present invention further provides the use of an agent according to the invention for determining the oncogenic property of an HCMV strain.

The present invention further provides the use of an agent according to the invention for determining the risk of carcinogenesis or for evaluating a cancer prognosis in an individual.

The agent implemented in the uses and methods according to the invention may be any agent that binds partially or completely to an amino acid sequence of formula (I) of the IE1 protein from HCMV, as described throughout the present specification.

In another embodiment, the agent implemented in the uses and methods according to the invention may be a nucleic acid sequence that binds partially or completely to a nucleic acid sequence that codes for the amino acid sequence of formula (I) of the IE1 protein from HCMV, as described throughout the present specification.

Therefore, the present invention refers to the use of a nucleic acid sequence that binds partially or completely to a nucleic acid sequence that codes for the amino acid sequence of formula (I) of the IE1 protein from HCMV for determining the risk of carcinogenesis or for evaluating a cancer prognosis in an individual.

It further relates to the use of a nucleic acid sequence that binds partially or completely to a nucleic acid sequence that codes for the amino acid sequence of formula (I) of the IE1 protein from HCMV, for determining the oncogenic property of an HCMV strain.

A nucleic acid sequence according to the invention may be an ADN or an ARN sequence.

To detect or quantify the presence of DNA or RNA, methods of marking nucleic acids can include the use of fluorescent dyes, radioactive isotopes, biotinylated probes, or enzyme-conjugated probes, which can then be detected through techniques such as PCR, qPCR, RT-PCR, ELISA-PCR, Northern blotting, fluorescence in situ hybridization (FISH), or hybridization assays.

According to a particular embodiment, the DNA or ARN sequence may be linked to at least one marker.

Markers that bay be used according to the invention include fluorescent dyes, such as SYBR Green, Hoechst 33258, Ethidium Bromide (EtBr), Fluorescein isothiocyanate (FITC), Cy3, and Cy5; radioactive isotopes such as 32P (Phosphorus-32) and 35S (Sulfur-35); biotinylated probes; and enzyme-conjugates probes such as Alkaline Phosphatase and Horseradish Peroxidase (HRP).

A nucleic acid sequence encoding the amino acid sequence of formula (I) of the IE1 protein from HCMV as defined throughout the specification may be determined by one skilled in the art using the genetic code, taking into account the redundancy of the genetic code.

The genetic code is a set of rules by which nucleotide sequences in DNA or RNA are translated into amino acid sequences in proteins, where each codon (three-nucleotide sequence) specifies an amino acid. A given amino acid may be coded by different triplets, or codons, due to the redundancy of the genetic code, where multiple codons can encode the same amino acid, often differing only in the third nucleotide of the codon.

According to a particular embodiment, a nucleic acid sequence encoding the amino acid sequence of formula (I) of the IE1 protein from HCMV comprises a nucleic acid sequence having at least 80 %, in particular at least 90 %, more particularly had 100 % sequence identity with the nucleic acid sequence set forth as SEQ ID NO: 26.

In a particular embodiment, a nucleic acid sequence that codes for the amino acid sequence of formula (I) of the IE1 protein from HCMV consists of the nucleic acid sequence set forth as SEQ ID NO: 26.

In a particular embodiment, the nucleic acid sequence that binds partially or completely to a nucleic acid sequence that codes for the amino acid sequence of formula (I) of the IE1 protein from HCMV is a primer.

Primer according to the present invention can be easily determined by one skilled in the art. Examples of such primers include, but are not limited to, nucleic acid sequences set forth as SEQ ID NO: 27 and SEQ ID NO: 28.

As used herein, *"the risk of carcinogenesis"* in an individual refers to the likelihood or potential for a substance, exposure, or environmental factor to cause cancer by inducing changes in the genetic material of cells, leading to uncontrolled cell growth and tumor formation.

A *"cancer prognosis"* refers herein to the likely course and outcome of the disease, including the chances of recovery, progression, or recurrence, based on factors such as the type and stage of cancer, the patient's overall health, and response to treatment. It provides an estimate of survival or life expectancy, though it can vary widely between individuals.

The present invention relates to a method for evaluating the oncogenic property of an HCMV strain, comprising at least the steps of :
i) determining a presence or a level of expression or activity of an amino acid sequence of formula (I) of the IE1 protein from the HCMV strain :

   LX₁X₂X₃E (I)

   wherein L is a leucine;
   X₁, X₂ and X₃ are, independently, any naturally occurring amino acid, in particular X₂ is a threonine; and
   E is a glutamic acid,
   in an HCMV strain; and/or
ii) detecting a presence or measuring an amount of a nucleic acid sequence encoding the amino acid sequence of formula (I) of the IE1 protein from the HCMV strain :

   LX₁X₂X₃E (I)

   wherein L is a leucine;
   X₁, X₂ and X₃ are, independently, any naturally occurring amino acid, in particular X₂ is a threonine; and
   E is a glutamic acid,
   in an HCMV strain. also Indeed, the invention provides a method for evaluating the oncogenic property of an HCMV strain, comprising at least the step of determining a presence or a level of expression or activity of an amino acid sequence of formula (I) of the IE1 protein from the HCMV strain :

      LX₁X₂X₃E (I)
   wherein L is a leucine;
   X₁, X₂ and X₃ are, independently, any naturally occurring amino acid, in particular X₂ is a threonine; and
   E is a glutamic acid,
   in an HCMV strain.

By *"determining the activity"* of an amino acid sequence, it is understood in the present text, determining the direct or indirect binding activity of the amino acid sequence to at least one of its targets. For example, determining the activity of an amino acid sequence of formula (I) of the IE1 protein from the HCMV strain includes determining its binding activity to proteins p53 and/or Rb.

p53 and Rb binding activity to IE1 can be measured using a protein-protein interaction assay such as by co-immunoprecipitation and western blotting, yeast two-hybrid systems, protein-fragment complementation assays (PCA), affinity purification/mass spectrometry, protein microarrays, fluorescence resonance energy transfer (FRET), and Microscale Thermophoresis (MST).

According to a particular embodiment, the oncogenic property of the HCMV strain is determined by comparing the level of expression or activity an amino acid sequence of formula (I) of the IE1 protein to a reference threshold value.

According to a particular embodiment, the method further comprises at least a step of comparing the level of expression or activity an amino acid sequence of formula (I) of the IE1 protein to a reference threshold value.

In the context of a method for evaluating the oncogenic property of an HCMV strain according to the invention, a *"reference threshold value"* refers to a predetermined level of expression or activity of the amino acid sequence of formula (I) of the IE1 protein, established based on experimental data, statistical analysis, or comparative studies with known oncogenic and non-oncogenic HCMV strains.

This threshold may correspond to an absolute copy number, a relative expression level, or a normalized ratio, depending on the detection method used. The oncogenic potential of an HCMV strain can be determined by comparing the detected amount of the amino acid sequence of formula (I) of the IE1 protein to this reference threshold value, wherein values above or below the threshold indicate a higher or lower oncogenic potential, respectively.

For example, a negative control that can be used a threshold of the invention can be obtained by using a non-oncogenic HCMV strain, such as the TB40-F strains, or a heatinactivated HCMV strain, for example, 100°C for 10 minutes. A positive control that can be used as a threshold of the invention can be obtained by using an oncogenic HCMV strain, such as the HCMV-DB strain (Kumar et al., eBioMedicine, Volume 30, April 2018, Pages 167-183) and HCMV-BL strain (Nehme et al., Oncogene, volume 40, pages 3030-3046 (2021)).

The present invention also provides a method for evaluating the oncogenic property of an HCMV strain, comprising at least the step of detecting a presence or measuring an amount of a nucleic acid sequence encoding the amino acid sequence of formula (I) of the IE1 protein from the HCMV strain :

LX₁X₂X₃E (I)

wherein L is a leucine;
X₁, X₂ and X₃ are, independently, any naturally occurring amino acid, in particular X₂ is a threonine; and
E is a glutamic acid,
in an HCMV strain.

The detection of a nucleic acid sequence encoding the amino acid sequence of formula (I) of the IE1 protein in the genome of an HCMV strain can be performed using any method known to those skilled in the art. Such methods include, but are not limited to, polymerase chain reaction (PCR), quantitative PCR (qPCR), reverse transcriptase PCR (RT-PCR), ELISA-PCR, digital droplet PCR (ddPCR), nucleic acid hybridization techniques (such as Southern blot or in situ hybridization), next-generation sequencing (NGS), Sanger sequencing, or other molecular diagnostic approaches. The choice of method may depend on factors such as sensitivity, specificity, and available sample material.

In a particular embodiment, detecting the presence of a nucleic acid sequence encoding the amino acid sequence of formula (I) of the IE1 protein comprises amplifying a nucleic acid sample from the HCMV strain using polymerase chain reaction (PCR) with primers specific for the nucleic acid sequence encoding the amino acid sequence of formula (I) of the IE1 protein.

Such primers can be easily determined by one skilled in the art. Examples of such primers include, but are not limited to, nucleic acid sequences set forth as SEQ ID NO: 27 and SEQ ID NO: 28.

According to a particular embodiment, the oncogenic property of the HCMV strain is determined by comparing the amount of a nucleic acid sequence encoding the amino acid sequence of formula (I) of the IE1 protein to a reference threshold value.

According to a particular embodiment, the method further comprises a step of comparing the amount of a nucleic acid sequence encoding the amino acid sequence of formula (I) of the IE1 protein to a reference threshold value.

In the context of a method for evaluating the oncogenic property of an HCMV strain according to the invention, a *"reference threshold value"* refers to a predetermined amount of nucleic acid sequence encoding the amino acid sequence of formula (I) of the IE1 protein, established based on experimental data, statistical analysis, or comparative studies with known oncogenic and non-oncogenic HCMV strains.

This threshold may correspond to an absolute copy number, a relative expression level, or a normalized ratio, depending on the detection method used. The oncogenic potential of an HCMV strain can be determined by comparing the detected amount of the amino acid sequence of formula (I) of the IE1 protein to this reference threshold value, wherein values above or below the threshold indicate a higher or lower oncogenic potential, respectively.

The invention further relates to a method for determining the risk of carcinogenesis in an individual or for evaluating a cancer prognosis in an individual comprising at least the steps of:
i) determining, in an isolated biological sample, obtained from said individual, a presence or a level of expression or activity of an amino acid sequence of formula (I) of the IE1 protein from an HCMV strain :

   LX₁X₂X₃E (I)

   wherein L is a leucine;
   X₁, X₂ and X₃ are, independently, any naturally occurring amino acid, in particular X₂ is a threonine; and
   E is a glutamic acid ; and/or
ii) detecting a presence or measuring an amount of a nucleic acid sequence encoding the amino acid sequence of formula (I) of the IE1 protein from the HCMV strain:

   LX₁X₂X₃E (I)

   wherein L is a leucine;
   X₁, X₂ and X₃ are, independently, any naturally occurring amino acid, in particular X₂ is a threonine; and
   E is a glutamic acid,
      in an isolated biological sample from said individual.

Indeed, the invention provides a method for determining the risk of carcinogenesis in an individual or for evaluating a cancer prognosis in an individual comprising at least the steps of determining, in an isolated biological sample, obtained from said individual, a presence or a level of expression or activity of an amino acid sequence of formula (I) of the IE1 protein from an HCMV strain :

LX₁X₂X₃E (I)

wherein L is a leucine;
X₁, X₂ and X₃ are, independently, any naturally occurring amino acid, in particular X₂ is a threonine; and
E is a glutamic acid.

In a particular embodiment, the method further comprises at least a step of comparing the level of expression or activity an amino acid sequence of formula (I) of the IE1 protein measured in the isolated biological sample from the individual to a reference threshold value.

In the context of a method for determining the risk of carcinogenesis in an individual or for evaluating a cancer prognosis in an individual according to the invention, a "*reference threshold value"* refers to a predetermined level of expression or activity of the amino acid sequence of formula (I) of the IE1 protein measured in a biological sample of an individual that does not suffer from a cancer expressing the IE1 protein from HCMV, in particular that does not suffer from a cancer expressing an amino acid sequence of formula (I) of the IE1 protein from an HCMV strain.

In a particular embodiment, a "*reference threshold value"* used throughout the specification may serve as a positive control. In such an embodiment, the "*reference threshold value"* refers to a predetermined level of expression or activity of the amino acid sequence of formula (I) of the IE1 protein measured in a biological sample of an individual that suffers from a cancer expressing the IE1 protein from HCMV, in particular that suffers from a cancer expressing an amino acid sequence of formula (I) of the IE1 protein from an HCMV strain.

The invention further provides a method for determining the risk of carcinogenesis in an individual or for evaluating a cancer prognosis in an individual comprising at least the steps of detecting a presence or measuring an amount of a nucleic acid sequence encoding the amino acid sequence of formula (I) of the IE1 protein from the HCMV strain:

LX₁X₂X₃E (I)

wherein L is a leucine;
X₁, X₂ and X₃ are, independently, any naturally occurring amino acid, in particular X₂ is a threonine; and
E is a glutamic acid
in an isolated biological sample from said individual.

In a particular embodiment, the method further comprises at least a step of comparing the amount of a nucleic acid sequence encoding the amino acid sequence of formula (I) of the IE1 protein measured in the isolated biological sample from the individual to a reference threshold value.

In the context of a method for determining the risk of carcinogenesis in an individual or for evaluating a cancer prognosis in an individual according to the invention, a "*reference threshold value"* refers to a predetermined amount of a nucleic acid sequence encoding the amino acid sequence of formula (I) of the IE1 protein measured in a biological sample of an individual that does not suffer from a cancer expressing the IE1 protein from HCMV, in particular that does not suffer from a cancer expressing an amino acid sequence of formula (I) of the IE1 protein from an HCMV strain.

According to a particular embodiment of any of the methods described above, the amino acid sequence of the IE1 protein from an HCMV strain is that of formula (II).

The amino acid sequences of formula (I) and formula (II) are defined throughout the specification.

In a particular embodiment, the amino acid sequence of formula (I) has the amino acid sequence set forth as SEQ ID NO: 20. In a particular embodiment, the amino acid sequence of formula (II) has the amino acid sequence set forth as SEQ ID NO: 21.

According to a particular embodiment, X₁, X₂, X₃ and, if present, X₆ and X₇, are, independently, selected from the group consisting of a serine, a lysine, a threonine, a phenylalanine and a glutamine.

In a particular embodiment, X₁ is a lysine; and/or X₂ is a threonine; and/or X₃ is a phenylalanine; and/or, if present, X₆ is a serine; and/or, if present, X₇ is a glutamine.

More particularly, X₁ is a lysine, X₂ is a threonine, X₃ is a phenylalanine, and, if present, X₆ is a serine and, if present, X₇ is a glutamine.

According to a particular embodiment, the amino acid sequence of formula (I) is the amino acid sequence LKTFE set forth as SEQ ID NO: 22.

According to a particular embodiment, the amino acid sequence of formula (II) is the amino acid sequence EESLKTFEQV set forth as SEQ ID NO: 23.

According to a particular embodiment, the amino acid sequence is the sequence set forth as SEQ ID NO: 24 (EESLKTFEQVTEDCNENPELDVL).

A nucleic acid sequence encoding the amino acid sequence of formula (I) of the IE1 protein from HCMV as used in any of the methods according to the invention may be determined by one skilled in the art using the genetic code, taking into account the redundancy of the genetic code.

The genetic code is a set of rules by which nucleotide sequences in DNA or RNA are translated into amino acid sequences in proteins, where each codon (three-nucleotide sequence) specifies an amino acid. A given amino acid may be coded by different triplets, or codons, due to the redundancy of the genetic code, where multiple codons can encode the same amino acid, often differing only in the third nucleotide of the codon.

According to a particular embodiment, a nucleic acid sequence encoding the amino acid sequence of formula (I) of the IE1 protein from HCMV comprises a nucleic acid sequence having at least 80 %, in particular at least 90 %, more particularly had 100 % sequence identity with the nucleic acid sequence set forth as SEQ ID NO: 26.

In a particular embodiment, a nucleic acid sequence that codes for the amino acid sequence of formula (I) of the IE1 protein from HCMV consists of the nucleic acid sequence set forth as SEQ ID NO: 26.

As used herein, "*a biological sample"* refers to any sample taken from a living organism, typically for the purpose of analysis or diagnosis. Biological samples as used herein can include blood, urine, saliva, tissue samples, bronchoalveolar lavages, breast colostrum, breast milk, sputum, cerebrospinal fluid (CSF), semen, fecal samples, and cells from a swab.

The determination of the presence, expression level, or activity of at least one amino acid sequence can be performed using a method selected from Western blotting, immuno-ELISA, fluorescence microscopy, or flow cytometry. The determination of the presence, expression level, or activity of at least one amino acid sequence can be based on the functional or antigenic characteristics of the sequence.

A determination can involve staining cells or histological sections, performed according to conventional methods. Alternatively, the determination of the presence, expression level, or activity of at least one amino acid sequence of the invention may include extracting a sequence from a cell or tissue sample, followed by binding with a specific labeled probe, such as an antibody, particularly an antibody of the invention, and detecting the probe. The marker can be a radioisotope, a fluorescent compound, an enzyme, or an enzymatic substrate.

According to one embodiment, the determination of the presence, expression level, or activity of at least one amino acid sequence of the invention can be performed by gel electrophoresis or column chromatography. A determination can be performed using a Western blot type method, which includes gel migration in one or two dimensions, possibly followed by a transfer to a blotting membrane, and detection of the amino acid sequence with an entity capable of specifically binding to it and being detected.

The determination of the presence, expression level, or activity of at least one amino acid sequence of the invention can be performed using methods involving affinity binding between an entity, such as an antibody, and the sequence to be detected. The entity can be added to a biological sample and incubated for a sufficient period of time to allow binding between the entity and the amino acid sequence, for example, for at least about 10 minutes. The entity can be labeled with a radioisotope, an enzyme, a fluorescent molecule, a chemiluminescent molecule, or any other marker suitable for direct detection.

Alternatively, a second entity, which has affinity for the first entity, such as a secondary antibody, can be used to amplify the signal. These reagents are well known in the field. For example, a primary antibody can be conjugated to biotin, and an avidin conjugated to horseradish peroxidase can be added as a second-step reagent. The final detection can use a substrate that undergoes a color change in the presence of peroxidase. A secondary antibody conjugated to a fluorescent marker or a radioisotope can also be used. The absence, presence, or quantification of the primary or secondary labeled entity can then be carried out using any known method in the field, such as flow cytometry, fluorescence microscopy, radiography, scintillation counting, etc.

Also, a classic sandwich-type method, such as ELISA, can be used. A sandwich-type method includes the binding of a first antibody, specific to an amino acid sequence of the invention to an insoluble support. Then the support carrying the antibody is incubated with a sample presumed to contain an amino acid sequence of the invention, followed by an incubation step in the presence of a second antibody, particularly an antibody of the invention, specific to the amino acid sequence bound to the first antibody. The incubation times should be sufficient to allow the desired binding, and can generally vary from 0.1 to 3 hours. Washing steps may be included between each incubation step with any of the aforementioned entities. The second antibody may carry a marker allowing its detection, such as a radioactive isotope, such as iodine (125I, 121I), carbon (14C), sulfur (35S), tritium (3H), indium (112In), an enzymatic marker such as glucose oxidase, a fluorescent marker like rhodamine, an affinity marker such as biotin, or chemiluminescent molecules.

In some embodiments, a method of the invention can be adapted for *in vivo* implementation. In these embodiments, a detectable labeled entity specific to an amino acid sequence of the invention, such as an antibody, is administered to an individual, for example by injection, and the labeled cells are located using imaging techniques, such as magnetic resonance imaging, computed tomography, etc.

Preferably, the determination of the presence, expression level, or activity of at least one amino acid sequence can be carried out by detecting the amino acid sequence or an antibody directed against this amino acid sequence.

A preferred method suitable for the invention can be an ELISA test, for example, using an antibody bound to a solid support, such as a polystyrene microtiter plate or nitrocellulose paper.

An antibody used to detect the amino acid sequence of the invention can be an antibody of the invention labeled with a radioactive isotope, such as iodine (125I, 121I), carbon (14C), sulfur (35S), tritium (3H), indium (112In), an enzymatic marker such as glucose oxidase, a fluorescent marker such as rhodamine, or an affinity marker such as biotin.

The detection of a presence or the measurement of an amount of a nucleic acid sequence encoding the amino acid sequence of formula (I) of the IE1 protein from the HCMV strain can be performed using a method selected from PCR, quantitative PCR (qPCR), reversetranscriptase PCR (RT-PCR), ELISA-PCR, Northern blotting, or fluorescence in situ hybridization (FISH). The detection of the presence or the measurement of the quantity of at least one nucleic acid sequence can be based on the hybridization or amplification characteristics of the sequence.

PCR is a widely used technique that amplifies a specific segment of DNA. It uses short DNA primers complementary to the target sequence, a heat-stable DNA polymerase (like Taq polymerase), and repeated cycles of denaturation, annealing, and extension to exponentially replicate the DNA segment. PCR is highly sensitive and can detect low amounts of nucleic acids. It's particularly useful for identifying specific sequences, mutations, or genes.

qPCR (also known as real-time PCR) is a variation of PCR that allows for the quantification of DNA or RNA in real-time during amplification. This method uses fluorescent dyes or probes that emit light when bound to the target nucleic acid, enabling real-time measurement of the amount of amplified product. qPCR is commonly used to measure gene expression levels, detect viral load, or quantify nucleic acid targets with high precision.

RT-PCR is a technique combining reverse transcription of RNA into cDNA and amplification of specific DNA targets using PCR. It is primarily used to measure the amount of a specific RNA.

Polymerase chain reaction enzyme-linked immunosorbent assay (or ELISA-PCR) is an immunodetection method that allows quantification of the PCR product directly after immobilization of the biotinylated DNA on a microplate.

Northern blotting is used to detect and characterize RNA sequences. The method involves separating RNA samples by size via gel electrophoresis, transferring them to a membrane, and then hybridizing the membrane with a labeled complementary probe (a sequence of DNA or RNA that is complementary to the target RNA). This technique can be used to assess gene expression levels, RNA processing (e.g., splicing), or to detect specific RNA isoforms.

FISH is a cytogenetic technique used to detect and localize the presence of specific nucleic acid sequences within intact cells or tissues. A fluorescently labeled probe, which is complementary to the target nucleic acid sequence, binds to its target within the cells or tissue section. The labeled probe can be detected under a fluorescence microscope, allowing for visualization and quantification of the nucleic acid in its natural context. FISH is often used for chromosome mapping, gene localization, or detection of specific RNA or DNA sequences in cells.

The present invention further provides a diagnostic kit comprising at least one agent according to the invention.

The agent included in the diagnostic kit according to the invention may be any agent that binds partially or completely to an amino acid sequence of formula (I) of the IE1 protein from HCMV, as described throughout the present specification.

In another embodiment, the agent included in the diagnostic kit according to the invention may be a nucleic acid sequence that binds partially or completely to a nucleic acid sequence that codes for the amino acid sequence of formula (I) of the IE1 protein from HCMV, as described throughout the present specification.

Therefore, the invention further relates to a diagnostic kit comprising at least one nucleic acid sequence that binds partially or completely to a nucleic acid sequence that codes for the amino acid sequence of formula (I) of the IE1 protein from HCMV.

A nucleic acid sequence according to the invention may be an ADN or an ARN sequence.

A nucleic acid sequence encoding the amino acid sequence of formula (I) of the IE1 protein from HCMV as defined throughout the specification may be determined by one skilled in the art using the genetic code, taking into account the redundancy of the genetic code.

The genetic code is a set of rules by which nucleotide sequences in DNA or RNA are translated into amino acid sequences in proteins, where each codon (three-nucleotide sequence) specifies an amino acid. A given amino acid may be coded by different triplets, or codons, due to the redundancy of the genetic code, where multiple codons can encode the same amino acid, often differing only in the third nucleotide of the codon.

According to a particular embodiment, a nucleic acid sequence encoding the amino acid sequence of formula (I) of the IE1 protein from HCMV comprises a nucleic acid sequence having at least 80 %, in particular at least 90 %, more particularly had 100 % sequence identity with the nucleic acid sequence set forth as SEQ ID NO: 26.

In a particular embodiment, a nucleic acid sequence that codes for the amino acid sequence of formula (I) of the IE1 protein from HCMV consists of the nucleic acid sequence set forth as SEQ ID NO: 26.

According to a particular embodiment, a nucleic acid sequence that binds partially or completely to a nucleic acid sequence that codes for the amino acid sequence of formula (I) of the IE1 protein from HCMV, in particular that binds partially or completely to the nucleic acid sequence set forth as SEQ ID NO: 26, comprises a nucleic acid sequence having at least 80 %, in particular at least 90 %, more particularly had 100 % sequence identity with the nucleic acid sequence set forth as SEQ ID NO: 35. According to a particular embodiment, a nucleic acid sequence that binds partially or completely to a nucleic acid sequence that codes for the amino acid sequence of formula (I) of the IE1 protein from HCMV consists of the nucleic acid sequence set forth as SEQ ID NO: 35.

In a particular embodiment, the at least one agent is a primer or a probe.

Primers and probes according to the present invention can be easily determined by one skilled in the art. Examples of such primers and probes include, but are not limited to, primers and probes comprising the nucleic acid sequence set forth as SEQ ID NO: 27 and SEQ ID NO: 28.

According to a particular embodiment, the diagnostic kit may further comprise means of detecting the presence and/or quantity of the agent in a biological sample *in vitro* or *ex vivo.*

A biological sample can be as defined above.

Means for detecting the presence and/or quantity of the agent in a biological sample are well known to one skilled in the art.

Examples of such means include immunoassay methods, fluorescence-based techniques, mass spectrometry, PCR-based methods, imaging-based detection, biosensors and electrochemical sensors.

According to a particular embodiment the means to detect the presence and/or quantity of the agent can be an immunoassay method. For instance, ELISA (Enzyme-Linked Immunosorbent Assay) can be used, where the agent, such as an antibody or peptide, is detected by binding to a specific antibody or antigen. This technique is capable of quantifying the agent by producing a color change or fluorescence when a substrate reacts with an enzyme linked to the antibody. Another method within this category is Western blotting, where proteins or peptides are separated on a gel, transferred to a membrane, and detected using specific antibodies or probes that bind to the agent.

For detection involving fluorescence-based techniques, fluorescence microscopy is a widely used approach. This allows *in vitro, in vivo* or *ex vivo* observation of agents that are labeled with fluorescent markers, enabling both qualitative and quantitative detection. Fluorescence-Activated Cell Sorting (FACS) is another technique in which flow cytometry is utilized to detect the presence and quantify agents like antibodies or aptamers that have been tagged with fluorescent dyes.

Another highly sensitive detection method is radioisotope labeling. In this approach, agents such as small inhibiting molecules or antibodies are labeled with radioactive isotopes like iodine-125, carbon-14, or tritium. These radiolabeled agents can then be detected using scintillation counting or specialized imaging techniques, providing precise quantification and localization within biological samples.

Mass spectrometry is also a powerful tool for detection, particularly for analyzing and quantifying molecular components. Techniques like LC-MS (Liquid Chromatography-Mass Spectrometry) can be used to measure the molecular weight and structure of the agent, offering detailed information on its presence and concentration in a sample.

In addition to these methods, Surface Plasmon Resonance (SPR) provides real-time monitoring of molecular interactions. This technique can be used to measure the binding of the agent to its target (such as peptides, proteins, or antibodies) immobilized on a sensor surface, providing insights into the presence and kinetics of the agent's binding.

PCR-based methods are also useful for detecting and quantifying agents in biological samples. qPCR (Quantitative Polymerase Chain Reaction), for example, can be used to quantify nucleic acids related to the agent, such as aptamers or small interfering RNAs, or to measure gene expression. RT-PCR and ELISA-PCR, described elsewhere, are also useful for detecting and quantifying nucleic acid sequences in a kit according to the invention.

For imaging-based detection, MRI (Magnetic Resonance Imaging) is frequently used in vivo. It allows for tracking agents that have been labeled with magnetic particles or other detectable tracers. PET (Positron Emission Tomography) is another advanced imaging technique, especially useful for tracking radioactive tracers that have been incorporated into agents, helping visualize their distribution within living organisms.

Biosensors like Surface-Enhanced Raman Spectroscopy (SERS) can provide sensitive detection of small molecules or agents by measuring the inelastic scattering of light. This method is particularly useful for detecting low concentrations of agents with high precision.

Finally, electrochemical sensors offer a way to detect small molecules or agents through changes in electrical signals when the agent interacts with a surface or electrode. This approach is often employed for its simplicity and sensitivity, particularly in point-of-care applications.

According to a particular embodiment, the at least one nucleic acid sequence that binds partially or completely to a nucleic acid sequence that encodes the amino acid sequence of formula (I) of the IE1 protein from HCMV that is further linked to a marker.

Markers that may be used according to the invention include fluorescent dyes, such as SYBR Green, Hoechst 33258, Ethidium Bromide (EtBr), Fluorescein isothiocyanate (FITC), Cy3, and Cy5; radioactive isotopes such as 32P (Phosphorus-32) and 35S (Sulfur-35); biotinylated probes; and enzyme-conjugates probes such as Alkaline Phosphatase and Horseradish Peroxidase (HRP).

According to a particular embodiment, the diagnostic kit comprising a nucleic acid sequence that binds partially or completely to a nucleic acid sequence that encodes the amino acid sequence of formula (I) of the IE1 protein from HCMV may further comprise reagents for the amplification or hybridization of said nucleic acid sequence.

As used herein, *"reagents"* refer to the substances or chemicals included in the diagnostic kit that are used to facilitate the amplification (increasing the number of copies) or hybridization (binding of complementary nucleic acid strands) of a nucleic acid sequence. These reagents are necessary for performing specific biochemical reactions in molecular diagnostics.

Reagents for the amplification of a nucleic acid sequence may include DNA polymerase (such as Taq polymerase or Pfu polymerase), which is an enzyme that synthesizes new DNA strands, nucleotides (dNTPs), which are the building blocks used to form the new DNA strands, and buffer solutions that maintain optimal conditions for polymerase activity. Magnesium chloride (MgCl₂) is another important reagent, as it acts as a cofactor for DNA polymerase during amplification. While not a reagent itself, a thermal cycler is also used to control the temperature cycles required for the amplification process.

Reagents for the hybridisation of a nucleic acid sequence may include denaturation reagents, such as sodium hydroxide (NaOH), which are used to separate double-stranded DNA into single strands; salt solutions like SSC or SSPE that are crucial to maintain ionic strength and help the probe bind to the target sequence; blocking agents, such as bovine serum albumin (BSA) or salmon sperm DNA, to prevent non-specific binding during hybridization; wash buffers, including those with lower salt concentrations for higher specificity, which help remove non-specific bindings after hybridization; and labeled detection reagents like enzyme-conjugated antibodies or fluorescent dyes that are used to detect the presence of bound probes.

The reagents according to the invention may serve in techniques such as PCR, qPCR, RT-PCR, ELISA-PCR, Northern blotting, fluorescence in situ hybridization (FISH), or hybridization assays. These techniques are known in the art and further described in the present specification.

### EXAMPLES

### Material and methods

### Cell Cultures

Human ovarian surface epithelial cells (HOSE cells or OECs) and primary human astrocytes (HAs) were purchased from Innoprot (Derio, Spain). Human mammary epithelial cells (HMECs) were purchased from Life Technologies 3 (Carlsbad, CA, USA). OECs were cultivated in ovarian epithelial cell medium (serum-free) supplemented with ovarian epithelial cell growth supplement (OEpiCGS) and penicillin/streptomycin solution (P60132, Innoprot). HAs were cultivated in astrocytes medium (Innoprot), whereas HMECs were cultivated in HMECs medium (Life Technologies) supplemented with HMECs supplement and bovine pituitary extract (Life Technologies). Cells were cultured under standard conditions (37°C, 5% CO2, 95% humidity). To note that, HOSE cells or OECs were isolated from healthy human ovaries, as mentioned in the technical data sheet (P10982, Innoprot). Regular screenings were conducted monthly to confirm the absence of mycoplasma in the cultures. (VenorGem classic mycoplasma detection, Minerva Biolabs).

### Characterization of HCMV clinical isolates

The clinical HCMV strain HCMV-DB (GenBank KT959235) was isolated from a patient who was hospitalized at Besançon University Hospital (France) as previously described (Nehme et al., Oncogene 2021;40:3030-46 and Kumar et al., EBioMedicine 2018;30:167-83). The preparation of cell-free virus stocks and infections followed previously outlined procedures. A thorough screening of our viral stocks was undertaken to eliminate the possibility of other oncoviruses being present (Nehme et al., 2021).

### Viral growth and detection

For HCMV quantification, cell-free infectious supernatant was collected, DNA was isolated (EZNA Blood DNA Kit, D3392-02, Omega BIO-TEK, Norcross, GA) and conventional PCR as well as real-time IE1 quantitative PCR (qPCR) was performed using KAPA SYBR FAST Master Mix (KAPA BIOSYSTEMS, Potters Bar, UK) and IE1 primers. Real-time qPCR reactions were activated at 95°C for 10 minutes and then 50 cycles (15 seconds at 95°C and 1 minute at 60°C) were conducted using a Stratagene Mx3005P thermocycler (Agilent Technologies, Santa Clara, CA). Results collection and analysis were done using MxPro qPCR software. Primers used are listed in the Table 1 below:

**Table 1**

| **SEQ ID NO:** | **Primer** | **Primer sequence** |
|---|---|---|
| 1 | IE1-forward | CGACGTTCCTGCAGACTATG |
| 2 | IE1-reverse | TCCTCGGTCACTTGTTCAAA |
| 3 | EZH2-forward | TCGTGCCCTTGTGTGATAGC |
| 4 | EZH2-reverse | TCTCGGACAGCCAGGTAGC |
| 5 | MYC-forward | ACACCCTTCTCCCTTCG |
| 6 | MYC-reverse | CCGCTCCACATACAGTCC |
| 7 | SOX2-forward | GGGAAATGGAGGGGTGCAAAAGAGG |
| 8 | SOX2-reverse | TTGCGTGAGTGTGGATGGGATTGGTG |
| 9 | Nanog-forward | TCCTCCTCTTCCTCTATACTAAC |
| 10 | Nanog-reverse | CCCACAATCACAGGCATAG |
| 11 | Akt-forward | ATCCCCTCAACAACTTCTCAGT |
| 12 | Akt-reverse | CTTCCGTCCACTCTTCTCTTTC |
| 13 | β-2-Microglobulin-forward | GATGAGTATGCCTGCCGTGTG |
| 14 | β-2-Microglobulin-reverse | CAATCCAAATGCGGCATCT |

### Plasmid construction

The pLVX-IE1-DB WT was constructed from the pLVX vector as a parental plasmid. Briefly, the parental plasmid was digested by EcoRI and BamH1 (New England BioLabs; R3101S and R3136S). IE1-DB WT fragment was generated with specific primers using the pcDNA3.1-IE1-DB plasmid, and DNA polymerase Platinum SuperFI enzyme (Thermofisher; 12351010). Primers used are listed in the Table 2. PCR fragment was ligated into the digested parental plasmid using NEBuilder HiFi DNA Assembly (New England BioLabs; E2621). Afterward, the resulting plasmid was transformed into DH5-apha bacteria at 37°C. The plasmid was verified by digestion using the restriction enzyme Nco1 (New England BioLabs; R3193S) and by Sanger sequencing using specific primers that were listed in the Table 2 below:

**Table 2**

| **SEQ ID NO:** | **Primer** | **Sequence** |
|---|---|---|
| **Primers for cloning** | | |
| 15 | Lenti-pLVX-IE1-DB-EcoRI-forward | CTCTACTAGAGGATCTATTTCCGGTATGGAGTCCTCTGCCAAGAG |
| 16 | Lenti-pLVX-IE1-DB-BamHI-reverse | GGAGGGAGAGGGGCGGGATCCTATTACTGGTCAGCCTTGCT |

| **Primers for sequencing** | | |
|---|---|---|
| 17 | IRES-reverse | GCATTCCTTTGGCGAGAG |
| 18 | CMV-forward | CGCAAATGGGCGGTAGGCGTG |
| 19 | IE1-DB-nt3476-nt3495 | CCAATGGCTGCAGTCAGGCC |

The pLVX-IE1-DB mutated was constructed from pLVX-IE1-DB WT as a parental plasmid. Briefly, the parental plasmid was digested by BamHI and ClaI (New England Bio Labs; R3236S and R0197S). Two fragments were generated from the pLVX-IE1-DB WT to introduce the LKTFE to LKTAS mutation in IE1-DB with specific primers using DNA polymerase Platinum SuperFI enzyme (Thermofisher; 12351010). Primers are listed in Table 3 below. PCR fragments were ligated into the digested parental plasmid using NEBuilder HiFi DNA Assembly (New England BioLabs; E2621). Then the resulting plasmids were transformed into NEB^{®} Stable Competent E. coli (C3040H) at 30°C. Plasmids have been verified by digestion using the restriction enzyme Nco1 (New England BioLabs; R3193S) and by Sanger Sequencing using specific primers listed in Table 3.

**Table 3**

| **SEQ ID NO:** | **Primer** | **Sequence** |
|---|---|---|
| **Primers for cloning** | | |
| 29 | Lenti-pLVX-IE1-DB-mutated-PCR1-FW | CAGGGACAGCAGAGATCCAGTTTAT |
| 30 | Lenti-pLVX-IE1-DB-mutated-PCR1-RV | CTCGGTCACTTGTGAAGCAGTTTTGAGGGATTC |
| 31 | Lenti-pLVX-IE1-DB-mutated-PCR2-FW | GAATCCCTCAAAACTGCTTCACAAGTGACCGAG |

| **Primers for sequencing** | | |
|---|---|---|
| 32 | IRES-reverse | GCATTCCTTTGGCGAGAG |
| 33 | CMV-forward | CGCAAATGGGCGGTAGGCGTG |
| 34 | IE1-DB-nt3476-nt3495 | CCAATGGCTGCAGTCAGGCC |

### Lentiviral production and transduction

HEK293T cells were seeded at a density of 7×10⁶ cells per flask and grown in a 175 cm² flask. Twenty-four hours later, cells were transfected with the pLVX-IE1-DB WT plasmid (9 µg), pVSV-G (2.25 µg), and psPAX2 (6.75 µg) packaging vectors using the calcium phosphate transfection method according to the manufacturer's protocol (TaKaRa; Calphos 631312). Seventy-two hours post-transfection, the virus-containing supernatant (17 ml) was filtered (Steriflip, Merck; SCGP00525) before being concentrated by ultracentrifugation (100 000g for sixteen hours at 4°C) (Optima XPN, Beckman) and stored at -80°C. The concentrated viral particles were titrated by ELISA p24 (INNOTEST^{®} HIV Antigen mAb; 80563). The OECs, HAs and HMECs were transduced at MOI of 1500, 700, and 1500, respectively. Briefly, cells (1×10⁶ cells/well in 96-well plates) were infected with 30 µl of pLVX-IE1-DB/VSV-G pseudo-typed viruses containing 8µg/ml of polybrene (Santa Cruz; SC134220). Then, cells were resuspended and cultured at a final concentration of 1×10⁶ cells/ml. The selection of transduced cells was performed by adding 2µg/mL of puromycin to the culture media.

### Western Blotting

IE1, Rb, pRb, p53, Myc, EZH2, Nanog, SOX2, Vimentin, and E-cadherin expression in OECs-control (OECs-CTL) and IE1-DB-transduced OECs (CTO-IE1-DB), was assessed as described in El Baba et al. (Oncogene, 2023 Oct;42(41):3047-3061). β-actin was used as a loading control. Antibodies used are supplied Table 3 below:

**Table 3**

| **Antibody** | **Commercial reference** |
|---|---|
| Anti-Myc Tag | 06-549-25UG/Merck KGaA, (Darmstadt, Germany) |
| EZH2 | AB_2793397/Active Motif (Carlsbad, CA, USA) |
| Ki67Ag | BD-556026/BD Biosciences (Franklin Lakes, USA) |
| CMV pp72 (IE1) | SC-69834/Santa Cruz Biotechnology (CA, USA) |
| IE1 | ab53495/Abcam (Cambridge, UK) |
| SOX2 | ab97959/Abcam (Cambridge, UK) |
| Nanog | SC-293121/Santa Cruz Biotechnology (CA, USA) |
| Vimentin | SC-6260/Santa Cruz Biotechnology (CA, USA) |
| E-cadherin | SC-8426/Santa Cruz Biotechnology (CA, USA) |
| p53 | SC-47698/Santa Cruz Biotechnology (CA, USA) |
| Rb | SC-102/Santa Cruz Biotechnology (CA, USA) |
| pRb | SC-377528/Santa Cruz Biotechnology (CA, USA) |
| Phalloidine | ab235137/ Abcam (Cambridge, UK) |
| Nestin | SC-23927/Santa Cruz Biotechnology (CA, USA) |
| b-actin | A2228/Merck KGaA, (Darmstadt, Germany) |
| FITC-conjugated anti-mouse antibody | BD- 553399/BD Biosciences (Franklin Lakes, USA) |
| PE-conjugated anti-mouse antibody | BD-551436/BD Biosciences (Franklin Lakes, USA) |
| FITC-conjugated anti-rabbit antibody | ab6717/Abcam (Cambridge, UK) |
| FITC-conjugated Rat Anti-Mouse | BD-553443/BD Biosciences (Franklin Lakes, USA) |
| FITC Mouse IgG2a, κ Isotype Control | BD-553456/BD Biosciences (Franklin Lakes, USA) |
| Propidium Iodide | P3566/Life Technologies (Eugene, USA) |

### Flow cytometry analysis

Cells (1×10⁵) were collected from OECs-CTL and CTO-IE1-DB as well as HMECs-CTL and IE1-DB-transduced HMECs (CTH-IE1-DB). Cells were fixed, permeabilized, and stained as previously reported in Nehme et al. Cytofluorometric analysis was achieved using a BD LSRFortessa X-20 (BD Biosciences) flow cytometer. FACSDiva software (BD Biosciences) was used for data collection and analysis. The antibodies used are provided in Supplementary Table 2. For cell cycle analysis, OECs-CTL and CTO-IE1-DB were washed in 1X PBS, fixed in 70% ethanol, and resuspended in 50 µg/ml propidium iodide (P3566, life technologies, Eugene, USA) with 0.1 mg/ml RNase (R4642, Sigma-Aldrich, Saint-Louis, MO, USA), then incubated at 37°C for 30 min as described in Nehme et al. (eBioMedicine 2022;80:104056).

### Reverse transcription quantitative polymerase chain reaction (RT-qPCR)

The detection of IE1, EZH2, Myc, Sox2, Nanog, and hTERT transcripts was assessed by RT-qPCR. Briefly, total RNA was extracted using E.Z.N.A. Total RNA Kit I (Omega Bio-Tech, GA, USA), and reverse transcription was performed using the SuperScript IV First-Strand Synthesis kit (Invitrogen, Carlsbad, CA, USA). The expression of markers was measured by real-time qPCR using a KAPA SYBR FAST Master Mix (KAPA BIOSYSTEMS, KK4601) and specific primers according to the manufacturer's protocol. Primers used are listed in Table 1 above.

### Confocal Microscopy

Confocal microscopy of OECs, HMECs, and HAs-CTL as well as CTO, CTH, and CEGBCs-IE1-DB was performed as previously detailed (Nehme et al., 2021). The antibodies used are provided in Table 3 above.

### Soft agar colony formation assay

Colony formation in soft agar (Colorimetric assay, CB135; Cell Biolabs Inc., San Diego, CA) seeded with OECs and HMECs-CTL as well as CTO and CTH-IE1-DB was performed as previously described (Nehme et al., 2021).

### Spheroid formation assay

Spheroids of OECs and HAs (CTO and CEGBCs-IE1-DB) were prepared as described previously (Nehme et al., 2021 and Nehme et al., 2022). Single cells (1×10⁴) isolated by accutase were seeded in a serum-free OECs or HAs medium containing methylcellulose.

### Invasion assay

Collagen invasion assay: Collagen I (Corning, New York, NY) of 1 mg/ml concentration was prepared in 1X PBS with 7.2mM NaOH and 0.1% HCl was added (El Baba et al., Oncogene 2023;42:3047-61 and El Baba et al., Oncogene 2023;42:2031-45 ). Prepared CEGBCs spheroids were incubated on ice for 30 min and then separately selected, washed in PBS, and subsequently included in the collagen solution. After 1 hour at 37°C in a cell incubator, serum-free astrocytes medium including the diverse treatments was added.

### Statistical analysis

Quantitative results are reported as mean ± SD of independent experiments. Statistical analyses were done using Student's T test and Mann-Whitney test; a p-value≤0.05 was considered to be statistically significant [*: ≤0.05; **: ≤0.01; ***: ≤0.001]. Correlation analysis was done using Pearson correlation test. Microsoft Excel was used to construct the plots and histogram data.

### Example 1: Transduction of OECs using GFP, HCMV-IE1-DB wild-type and HCMV-IE1-DB mutated plasmids

The inventors constructed two plasmids: pHCMV-IE1-DB wt and pHCMV-IE1-DB mut as described above.

In the mutated plasmid, point mutations were introduced into the pHCMV-DB-IE1 plasmid, targeting the IE1 protein sequence. Specifically, these mutations involved the substitution of phenylalanine with alanine at position 66 and glutamic acid with serine at position 67, i.e. in the LX₁X₂X₃E amino acid sequence of HCMV IE1 protein.

The plasmids were then transduced in OECs as described above. A plasmid carrying the GFP gene was also transduced in a cell group and used as a control.

The inventors assessed the protein IE1 expression by western blot in OECs-GFP (OECs-CTL), OECs-IE1-DB wild type (CTOs-IE1-WT), and OECs-IE1-DB mutated (OECs-IE1-Mut) lysates with β-Actin serves as the loading control.

Results are provided in **Figure 1** showing the expression of IE1 protein mainly in OECs-IE1-Mut and CTOs-IE1-WT lysates; IE1 protein was not expressed in OECs-CTL. B actin was present in all samples showing the equal loading.

OECs transduced with the pHCMV-DB-IE1 plasmid generated CMV transformed ovarian cells or CTO-IE1-DB cells.

Microscopic images were captured for OECs transduced with GFP, pHCMV-DB-IE1-WT, and pHCMV-DB-IE1-Mut plasmids.

The inventors observed that the morphology of CTOs-IE1-WT cells was characterized by dense cell clusters (see **Figure 2**). On the contrary, no specific cellular morphologies were detected in the cell cultures of OECs-CTL and OECs-IE1-Mut.

Taken together, these results show that the IE1 protein mutated in its LX₁X₂X₃E amino acid sequence hindered the transformation of OECs into CTO-IE1-DB cells.

### Example 2: Tumor suppressor depletion and oncogenic properties of protein IE1

The inventors then studied the expression of tumor suppressors Rb and p53 as well as pRb in the different transduced cells. These proteins are known for their role in cell cycle regulation, tumor suppression, and apoptosis. p53 acts as a "guardian of the genome," halting the cell cycle in response to DNA damage by activating p21, which inhibits cyclin-dependent kinases, and inducing apoptosis if damage is irreparable. Rb (Retinoblastoma protein) controls the G1/S checkpoint by binding and inhibiting E2F, a transcription factor required for DNA replication. pRb (phosphorylated Rb) is the inactive form of Rb, which occurs when CDK4/6 phosphorylates Rb, releasing E2F and allowing the cell to enter S-phase. Loss of function in p53 or Rb leads to uncontrolled cell division and is implicated in many cancers.

Depletion of tumor suppressors (Rb and p53) was observed in CTOs-IE1-WT cells but not in OECs-IE1- Mut and OECs-CTL cultures.

Indeed, western blot analysis demonstrated low expression levels of Rb and p53 proteins in lysates from CTOs-IE1-WT compared to OECs-CTL and OECs-IE1-Mut. However, high levels of pRb protein was detected in CTOs-IE1-WT lysates in comparison with OECs-CTL and OECs-IE1-Mut (see **Figure 3**).

In conclusion, mutating the LX₁X₂X₃E amino acid sequence of protein IE1 restores the levels of Rb and p53, demonstrating that IE1 suppresses these tumor suppressor proteins. However, the decrease in pRb expression upon IE1 mutation indicates that IE1 plays a role in increasing pRb levels and probably favoring Rb degradation via the 26S proteasome. This suggests that while IE1 negatively affects Rb and p53, it increases pRb levels favouring Rb degradation, highlighting the interplay between IE1 and the Rb/p53 tumor suppressor pathways.

The inventors then studied the oncogenic and stemness properties of the transduced cells via the expression of proteins Myc and EZH2. Myc and EZH2 are oncogenic regulators that promote transformation and tumor progression in tumor cells. Myc, a transcription factor that drives cell proliferation, metabolism, and survival by activating genes involved in growth and inhibiting tumor suppressors, enhances protein synthesis and genomic instability, contributing to malignant transformation. EZH2, a core component of the Polycomb Repressive Complex 2 (PRC2), silences tumor suppressor genes through H3K27 methylation, promoting stemness, invasion, and resistance to therapy. In transformed cells, Myc often upregulates EZH2, reinforcing an aggressive cancer phenotype by sustaining uncontrolled proliferation and repressing differentiation pathways.

Immunoblotting data showed that Myc and EZH2 protein expression was elevated in CTOs-IE1-WT cells compared to OECs-CTL and OECs-IE1-Mut cells showing that the former cells exhibited oncogenic and stemness properties that were absent in OECs-IE1-Mut cells (see **Figure 4**).

Confocal imaging revealed Myc and EZH2 localization mainly in CTOs-IE1-WT cells with lower expression in OECs-CTL with nuclei counterstained using DAPI. This confirmed that high expression levels of Myc and EZH2 are observed only in transformed CTOs-IE1-WT cells (see **Figure 5**).

Images were captured at a magnification of ×63, with a scale bar of 10 µm.

Colony formation was assessed in soft agar seeded with OECs-GFP (CTL), CTOs-IE1-WT, and OECs-IE1-Mut cells. The resulting colonies were visualized under an inverted light microscope at a magnification of ×20.

The results showed that CTOs-IE1-WT cells displayed an embryonic stem cell-like phenotype, which was absent in OECs-IE1-Mut cells. This confirmed that the presence of IE1 induces an embryonic stem cell-like phenotype, confirming that IE1 plays a role in maintaining stemness or reprogramming features in these cells. In contrast, the absence of this phenotype in OECs-IE1-Mut cells indicates that the mutation disrupts IE1's ability to promote stem cell-like characteristics, highlighting its potential role in cellular plasticity and transformation (see **Figure 6**).

Finally, the inventors observed the expression of Nanog and SOX2 proteins in the transduced cells. Nanog and SOX2 are key transcription factors that maintain pluripotency and self-renewal in transduced cells. Nanog prevents differentiation by regulating genes involved in stemness, while SOX2 sustain the undifferentiated state and reprogram somatic cells. In transduced cells, their expression suggests a shift toward a stem cell-like phenotype, promoting proliferation, survival, and potential transformation. Their sustained activity is often linked to enhanced plasticity and resistance to differentiation signals, which can contribute to tumorigenesis in transformed cells.

Western blot analysis confirmed the expression of Nanog and SOX2 proteins mainly in CTOs-IE1-WT lysates; lower protein expression was detected in OECs-IE1-Mut cells and OECs-CTL (see **Figure 7**). This confirms that the higher expression of Nanog and SOX2 in CTOs-IE1-WT cells is due to the fact that IE1 promotes a stem cell-like phenotype by enhancing pluripotency-associated factors. The reduced expression of these proteins in OECs-IE1-Mut and OECs-CTL cells indicates that the mutation disrupts IE1's ability to induce or maintain stemness. This finding highlights the potential role of IE1 in cellular reprogramming and transformation.

Confocal microscopy revealed the expression of Nanog and SOX2 proteins mainly in OECs-IE1-WT cells compared to OECs-CTL, with nuclei counterstained using DAPI. Images were captured at a magnification of ×63, with a scale bar of 10 µm. This data confirmed the western blot results obtained above (see **Figure 8**).

Finally, through microscopic images, the inventors captured the formation of spheroids in the presence of methylcellulose within the CTOs-IE1-WT cultures, observed at a magnification of ×20. No spheroids were observed in OECs-CTL and OECs-IE1-Mut cultures. The formation of spheroids in CTOs-IE1-WT cultures shows that IE1 enhances cellular aggregation, self-renewal, or a stem-like phenotype. The absence of spheroid formation in OECs-CTL and OECs-IE1-Mut cultures indicates that the mutation disrupts this capability, highlighting the role of IE1 in promoting three-dimensional growth, stemness and potential tumor-like properties (see **Figure 9**).

Taken all together, these results show that mutating the LX₁X₂X₃E amino acid sequence of IE1 restores Rb and p53 levels but reduces pRb, demonstrating a critical role of IE1 in tumor suppressor regulation. IE1 enhances Myc and EZH2 expression, promoting oncogenic and stemness properties, while OECs-IE1-Mut lack these characteristics. Higher Nanog and SOX2 levels in CTOs-IE1-WT further confirm IE1's role in maintaining a stem cell-like phenotype, which is lost in OECs-IE1-Mut cells. Additionally, IE1 facilitates spheroid formation, indicating its role in self-renewal, cellular aggregation, and tumor-like properties, whereas this ability is absent in IE1-Mut and OECs-CTL cultures. These findings highlight IE1's involvement in cellular reprogramming, transformation, and tumor progression.

### Example 3 : EMT (Epithelial-Mesenchymal Transition) Characteristics of the transduced cells and Expression of Associated Proteins

Next, the inventors studied the exhibited characteristics of epithelial-to-mesenchymal transition (EMT) for the OECs-CTL and CTOs-IE1-WT, and OECs-IE1-Mut cells. These characteristics included vimentin and E-cadherin protein expression. Vimentin and E-cadherin are key regulators of cellular plasticity in oncogenesis, particularly through epithelial-mesenchymal transition (EMT) and its reverse process, mesenchymal-epithelial transition (MET). E-cadherin, an epithelial marker, maintains cell-cell adhesion and suppresses invasion, while vimentin, a mesenchymal marker, promotes motility and metastasis. During EMT, E-cadherin is downregulated and vimentin is upregulated, allowing cancer cells to gain stem-like properties, migrate, and invade. However, their plasticity enables MET, facilitating colonization at distant sites. This dynamic interplay enhances tumor progression, metastasis, and therapy resistance, making vimentin and E-cadherin critical targets in cancer treatment.

The results showed that CTOs-IE1-WT cells exhibited characteristics of EMT and MET. Confocal microscopy revealed vimentin protein expression mainly in CTOs-IE1-WT cells and to a much lesser extent in OECs-CTL with nuclei counterstained using DAPI. In CTOs-IE1-WT cultures, vimentin was expressed in both large and small cells, indicating heterogeneity in the cell population. Images were captured at a magnification of ×63, with a scale bar of 10 µm (see **Figure 10**).

E-cadherin expression was analysed in OECs cultures. Confocal microscopy revealed E-cadherin protein expression mainly in OECs-CTL and to a lesser extent in CTOs-IE1-WT cells, with nuclei counterstained using DAPI. Images were taken at a magnification of ×63, with a scale bar of 10 µm. In CTOs-IE1-WT cultures, arrows highlighted E-cadherin expression specifically in small cells, suggesting a distinct subpopulation within the culture (see **Figure 11**).

Finally, western blot analysis revealed the high expression of vimentin and low expression of E-cadherin in CTOs-IE1-WT cells unlike OECs-IE1-Mut cells (see **Figure 12**).

Taken together, these results show that CTOs-IE1-WT cells displayed features of EMT/MET, indicating that these cells exhibit a dynamic balance between epithelial-mesenchymal transition and mesenchymal-epithelial transition. The presence of both EMT and MET characteristics in CTOs-IE1-WT shows high cellular plasticity, enabling these cells to transition between migratory and adhesive states, which is crucial for tumor progression, metastasis, and potential therapy resistance.

### Example 4: Transduction of HMECs and oncogenic characteristics

The inventors next prepared HMECs (human mammary epithelial cells) transduced with the pHCMV-DB-IE1 plasmid and generated CMV transformed human mammary epithelial cells or CTH-IE1-WT cells.

Microscopic images of HMECs transduced with GFP, IE1-DB-WT, and IE1-DB-Mut plasmids were captured. In the CTH-IE1-WT cultures, black arrows indicated the presence of large and dense cells as well as cells filled with lipid droplets, suggesting morphological changes associated with transformation. These morphological changes are representative of dedifferentiation, a process commonly observed in cancer progression. Lipid droplet accumulation is often linked to altered metabolism, increased lipid storage, and energy production, supporting rapid proliferation and survival under stress conditions. Such features are also seen in aggressive tumors, therapy-resistant cancer cells, and stem-like cancer cells, highlighting their role in tumor growth, invasion, and metastasis. Cells in HMECs-CTL and HMECs-IE1-Mut cultures didn't reveal any specific morphological changes. These observations were made at a magnification of ×40 (see **Figure 13**).

IE1 protein expression was observed via confocal microscopy images of CTH-IE1-WT cells with nuclei counterstained using DAPI. Images were taken at a magnification of ×63, with a scale bar of 10 µm.

The confirmation of IE1 presence in transduced HMECs through confocal microscopy indicates that the IE1 protein was successfully expressed in HMECs. This validation confirms that the transduction process was effective and shows that IE1 influences cellular behavior, potentially contributing to oncogenic transformation, stemness, and EMT characteristics (see **Figure 14**).

Confocal imaging revealed the expression of vimentin and Myc in CTH-IE1-WT cells unlike HMECs-CTL, highlighting the distinct protein expression patterns associated with the oncogenic and EMT traits of CTH-IE1-WT cells. Nuclei were counterstained using DAPI; the images were captured at a magnification of ×63, with a scale bar of 10 µm, (see **Figure 15**). These results show that CTH-IE1-WT cells exhibited both oncogenic and EMT characteristics.

Finally, western blot analysis revealed the expression of Myc, vimentin, and Sox2 in lysates of CTH-IE1-WT cells. (see **Figure 16**). These results show that CTH-IE1-wt cells exhibited oncogenic, stemness, and EMT characteristics.

### Example 5: Transduction of HAs and characteristics of stem and invasive cells

The inventors next prepared HAs (Human Astrocytes) cultures transduced with the pHCMV-DB-IE1 plasmid and generated CMV-elicited glioblastoma cells or CEGBCS-IE1-DB cells.

Microscopic images were taken of these HAs transduced with GFP (HAs-CTL), IE1-DB-WT (CEGBCS-IE1-WT), and IE1-DB-Mut (HAs-IE1-mut) plasmids. Morphological features observed in CEGBCS-IE1-wt cells included large cells, TNT (tunneling nanotubes), and NPC (neural progenitor cells) cells. These morphological changes are representative of cellular transformation and increased plasticity. The large cells suggest alterations in cell size and metabolism often seen in oncogenic transformation. The presence of TNTs indicates enhanced intercellular communication and the exchange of materials between cells, which is commonly associated with tumor progression and metastasis. The appearance of NPCs implies cellular reprogramming, where cells acquire stem-like properties, contributing to increased self-renewal, invasion, and potential resistance to therapies. These morphological changes collectively reflect the acquisition of oncogenic and stem-like characteristics, typically observed in aggressive, transformed cancer cells.

The HAs-CTL and HAs-IE1-mut cultures showed no morphological changes; in addition, cell death was observed in long-term cultures. These images were captured at a magnification of ×40, providing detailed insights into the distinct cellular morphology (see **Figure 17**).

IE1 and nestin dual staining was also detected by confocal microscopy images. IE1 and nestin were concomitantly detected in CEGBCS-IE1-WT cells unlike HAs-CTL. Nuclei were counterstained using DAPI; these images were captured at a magnification of ×63, with a scale bar of 10 µm, providing a detailed view of IE1 and nestin localization (see **Figure 18**).

Through confocal microscopic images, the inventors assessed the expression of IE1 and nestin in single cells of HAs-CTL and spheroids of CEGBCS-IE1-WT cells. IE1 and nestin were detected only in spheroids of CEGBCS-IE1-WT cells and not in single cells of HAs-CTL. Nuclei were counterstained using DAPI; the images were captured at a magnification of ×63, with a scale bar of 10 µm (see **Figure 19**).

The results showed that the formation of IE1/nestin-positive spheroids in CEGBCs-IE1-WT cultures suggests that IE1 enhances cellular aggregation, self-renewal, or a stem-like phenotype in HAs. The absence of spheroid formation in HAs-CTL and HAs-IE1-Mut cultures indicates that the mutation disrupts this capability, highlighting the role of IE1 in promoting three-dimensional growth and potential tumor-like properties.

Additionally, a 3D invasion assay conducted over 14 days showed CEGBCS-IE1-DB spheroids embedded in type-1 collagen in the presence of HCl. Arrows highlighted the invasive behaviour of the cells as they migrated out of the spheroids. The results showed that CEGBCS-IE1-WT cells display invasive and tumorigenic characteristics. This behavior suggests that IE1 expression promotes 3D invasion, a hallmark of tumor progression and metastasis. The observed invasion indicates that these cells can break free from the spheroids and infiltrate the surrounding matrix, mimicking cancer cell migration. Overall, this assay underscores the aggressive, metastatic potential of CEGBCS-IE1-WT cells in a 3D setting. These images were captured at a magnification of ×200, with a scale bar of 50 µm (see **Figure 20**).

Microscopic images captured the generation of colonies in the presence of methylcellulose, highlighting the detection of transformed CEGBCS-IE1-WT cells. In contrast, HAs-CTL and HAs-IE1-mut cells didn't form colonies, with single cells observed in the cultures, even when methylcellulose was present. These images were taken at a magnification of ×20, providing a clear view of the colonies morphology.

These results translate the ability of CEGBCS-IE1-WT cells to form three-dimensional structures or spheroids, and colonies in methyl cellulose medium, indicating their potential for self-renewal, cellular aggregation, and tumor-like behavior. In contrast, the lack of spheroid formation and colonies appearance in methyl cellulose medium in HAs-CTL and HAs-IE1-mut cells indicates a loss of cellular transformation, cellular plasticity and stemness potential (see **Figure 21**).

Finally, western blot analysis assessed the expression of Myc, vimentin, and nestin in lysates from HAs-CTL, CEGBCs-IE1-WT, and HAs-IE1-Mut cells. The results showed that CEGBCS-IE1-WT cells expressed high protein levels of Myc, vimentin, and nestin compared to HAs-CTL and HAs-IE1-Mut cells, which is representative of oncogenic, stemness, and EMT characteristics of the former cell culture (see **Figure 22**).

### SEQUENCE LISTING

SEQ ID NO : 1 : IE1-forward primer
   cgacgttcctgcagactatg
SEQ ID NO: 2 : IE1-reverse primer
   tcctcggtcacttgttcaaa
SEQ ID NO: 3 : EZH2-forward primer
   tcgtgcccttgtgtgatagc
SEQ ID NO: 4 : EZH2-reverse primer
   tctcggacagccaggtage
SEQ ID NO: 5 : MYC-forward primer
   acacccttctcccttcg
SEQ ID NO: 6 : MYC-reverse primer
   ccgctccacatacagtcc
SEQ ID NO: 7 : SOX2-forward primer
   gggaaatggaggggtgcaaaagagg
SEQ ID NO: 8 : SOX2-reverse primer
   ttgcgtgagtgtggatgggattggtg
SEQ ID NO: 9 : Nanog-forward primer
   tcctcctcttcctctatactaac
SEQ ID NO: 10 : Nanog-reverse primer
   cccacaatcacaggcatag
SEQ ID NO: 11 : Akt-forward primer
   atcccctcaacaacttctcagt
SEQ ID NO: 12 : Akt-reverse primer
   cttccgtccactcttctctttc
SEQ ID NO: 13 : β-2-Microglobulin-forward primer
   gatgagtatgcctgccgtgtg
SEQ ID NO: 14 : β-2-Microglobulin-reverse primer
   caatccaaatgcggcatct
SEQ ID NO: 15 : Lenti-pLVX-IE1-DB-EcoRI-forward primer
   ctctactagaggatctatttccggtatggagtcctctgccaagag
SEQ ID NO: 16 : Lenti-pLVX-IE1-DB-BamHI-reverse primer
   ggagggagaggggcgggatcctattactggtcagccttgct
SEQ ID NO: 17 : IRES-reverse primer
   gcattcctttggcgagag
SEQ ID NO: 18 : CMV-forward primer
   cgcaaatgggcggtaggcgtg
SEQ ID NO: 19 : IE1-DB-nt3476-nt3495 primer
   ccaatggctgcagtcaggcc
SEQ ID NO: 20 : amino acid sequence of formula (I)
   LX₁X₂X₃E
SEQ ID NO: 21 : amino acid sequence of formula (II)
   X₄X₅X₆LX₁X₂X₃EX₇X₈
SEQ ID NO: 22 : amino acid sequence LKTFE
   LKTFE
SEQ ID NO: 23 : amino acid sequence EESLKTFEQV
   EESLKTFEQV
SEQ ID NO: 24 : amino acid sequence of EESLKTFEQVTEDCNENPELDVL
   EESLKTFEQVTEDCNENPELDVL
SEQ ID NO: 25 : wild type amino acid sequence of IE1 protein of HCMV-DB 
MESSAKRKMDPDNPDEGPSSKVPRPETPVTKATTFLQTMLRKEVNSQLSLGDPLFPEL
AEESLKTFEQVTEDCNENPEKDVLTELVKQIKVRVDMVRHRIKEHMLKKYTQTEEKF
TGAFNMMGGCLQNALDILDKVHEPFEEMKCIGLTMQSMYENYIVPEDKREMWMAC
IKELHDVSKGAANKLGGALQAKARAKKDELRRKMMYMCYRNIEFFTKNSAFPKTTN
GCSQAMAALQNLPQCSPDEIMAYAQKIFKILDEERDKVLTHIDHIFMDILTTCVETMC
NEYKVTSDACMMTMYGGISLLSEFCRVLCCYVLEETSVMLAKRPLITKPEVISVMKR
RIEEICMKVFAQYILGADPLRVCSPSVDDLRAIAEESDEEEAIAAYTLATAGASSSDSL
VSPPESPVPATIPLSSVIVAENSDQEESEQSDEEQEEGAQEEREDTVSVKSEPVSEIGEV
APEEEEDGAEEPTTSGGKSTHPMVTRSKADQ
SEQ ID NO: 26: nucleic acid sequence coding for the amino acid sequence of formula (I) of the IE1 protein from HCMV
   ttcaaaagttttgag
SEQ ID NO: 27: nucleic acid sequence forward IE1 primer
   cgacgttcctgcagactatg
SEQ ID NO: 28: nucleic acid sequence reverse IE1 primer
   tcctcggtcacttgttcaaa
SEQ ID NO: 29: Lenti-pLVX-IE1-DB-mutated-PCR1-FW primer cagggacagcagagatccagtttat
SEQ ID NO: 30: Lenti-pLVX-IE1-DB-mutated-PCR1-RV primer
   ctcggtcacttgtgaagcagttttgagggattc
SEQ ID NO: 31: Lenti-pLVX-IE1-DB-mutated-PCR2-FW primer
   gaatccctcaaaactgcttcacaagtgaccgag
SEQ ID NO: 32: IRES-reverse primer
   gcattcctttggcgagag
SEQ ID NO: 33: CMV-forward primer
   cgcaaatgggcggtaggcgtg
SEQ ID NO: 34: IE1-DB-nt3476-nt3495 primer
   ccaatggctgcagtcaggcc
SEQ ID NO: 35: nucleic acid sequence binding partially or completely the nucleic acid sequence
   ctcaaaacttttgaa

## Claims

1. An agent that binds partially or completely to an amino acid sequence of formula (I) of the IE1 protein from HCMV:
LX₁X₂X₃E (I)
wherein L is a leucine;
X₁, X₂ and X₃ are, independently, any naturally occurring amino acid, in particular X₂ is a threonine; and
E is a glutamic acid.

2. Agent according to claim 1, wherein the agent binds partially or completely to an amino acid sequence of formula (II) of the IE1 protein:
X₄X₅X₆LX₁X₂X₃EX₇X₈ (II)
wherein E is a glutamic acid;
L is a leucine;
X₁, X₂, X₃, X₆ and X₇ are, independently, any naturally occurring amino acid, in particular X₂ is a threonine;
X₈ is a naturally occurring hydrophobic amino acid, in particular is a valine or a leucine, more particularly is a valine; and
X₄ and X₅ are, independently, a naturally occurring acidic amino acid, in particular X₄ and X₅ are a glutamic acid.

3. Agent according to claim 1 or 2, wherein X₁, X₂, X₃ and, if present, X₆ and X₇, are, independently, selected from the group consisting of a serine, a lysine, a threonine, a phenylalanine and a glutamine.

4. Agent according to any one of the preceding claims, wherein X₁ is a lysine; and/or X₂ is a threonine; and/or X₃ is a phenylalanine; and/or, if present, X₆ is a serine; and/or, if present, X₇ is a glutamine,
in particular wherein X₁ is a lysine, X₂ is a threonine, X₃ is a phenylalanine, and, if present, X₆ is a serine and, if present, X₇ is a glutamine,
more particularly wherein X₁ is a lysine, X₂ is a threonine, X₃ is a phenylalanine, , and, if present, X₄ and X₅ are a glutamic acid; and, if present, X₆ is a serine and, if present, X₇ is a glutamine; and, if present, X₈ is a valine.

5. Agent according to any one of the preceding claims, selected from the group consisting of small inhibiting molecules, peptides, peptidomimetics, antibodies, antibody fragments, aptamers, macrocycles, molecular glues, synthetic derivatives of natural ligands, and nanobodies, in particular the agent is a small inhibiting molecule.

6. A pharmaceutical composition comprising an agent according to any one of claims 1 to 5 and a pharmaceutically acceptable carrier.

7. The pharmaceutical composition according to claim 6, wherein the pharmaceutical composition is a vaccine.

8. Pharmaceutical composition according to claims 6 or 7, wherein the pharmaceutical composition further comprises at least one anticancer drug different from the agent as defined in any one of claims 1 to 5, and/or comprises at least one chemotherapy treatment, and/or comprises at least one anti-CMV treatment.

9. An agent according to any one of claims 1 to 5 or a pharmaceutical composition according to any one of claims 6 to 8 for their use in the prevention and/or treatment of cancer.

10. An agent or a pharmaceutical composition for their use according to claim 9, wherein the cancer is a cancer that expresses the IE1 protein of HCMV, in particular the cancer is selected from the group consisting of glioblastoma, breast cancer, ovarian cancer and prostate cancer, more particularly the cancer is glioblastoma.

11. Use of an agent according to any one of claims 1 to 5 or of a nucleic acid sequence that binds partially or completely to a nucleic acid sequence that codes for the amino acid sequence of formula (I) of the IE1 protein from HCMV, for determining the oncogenic property of an HCMV strain.

12. Use of an agent according to any one of claims 1 to 5 or of a nucleic acid sequence that binds partially or completely to a nucleic acid sequence that codes for the amino acid sequence of formula (I) of the IE1 protein from HCMV for determining the risk of carcinogenesis or for evaluating a cancer prognosis in an individual.

13. A diagnostic kit comprising at least one agent according to any one of claims 1 to 5 or comprising at least one nucleic acid sequence that binds partially or completely to a nucleic acid sequence that codes for the amino acid sequence of formula (I) of the IE1 protein from HCMV.

14. The diagnostic kit according to claim 13, further comprising means of detecting the presence and/or quantity of the agent in a biological sample *in vitro* or *ex vivo,* or further comprising reagents for the amplification or hybridization of the nucleic acid sequence.

15. A method for evaluating the oncogenic property of an HCMV strain, comprising at least the steps of :
i) determining a presence or a level of expression or activity of an amino acid sequence of formula (I) of the IE1 protein from the HCMV strain :
LX₁X₂X₃E (I)
wherein L is a leucine;
X₁, X₂ and X₃ are, independently, any naturally occurring amino acid, in particular X₂ is a threonine; and
E is a glutamic acid,
in an HCMV strain; and/or
ii) detecting a presence or measuring an amount of a nucleic acid sequence encoding the amino acid sequence of formula (I) of the IE1 protein from the HCMV strain :
LX₁X₂X₃E (I)
wherein L is a leucine;
X₁, X₂ and X₃ are, independently, any naturally occurring amino acid, in particular X₂ is a threonine; and
E is a glutamic acid,
in an HCMV strain.

16. A method for determining the risk of carcinogenesis in an individual or for evaluating a cancer prognosis in an individual comprising at least the steps of:
i) determining, in an isolated biological sample, obtained from said individual, a presence or a level of expression or activity of an amino acid sequence of formula (I) of the IE1 protein from an HCMV strain :
LX₁X₂X₃E (I)
wherein L is a leucine;
X₁, X₂ and X₃ are, independently, any naturally occurring amino acid, in particular X₂ is a threonine; and
E is a glutamic acid ; and/or
ii) detecting a presence or measuring an amount of a nucleic acid sequence encoding the amino acid sequence of formula (I) of the IE1 protein from the HCMV strain:
LX₁X₂X₃E (I)
wherein L is a leucine;
X₁, X₂ and X₃ are, independently, any naturally occurring amino acid, in particular X₂ is a threonine; and
E is a glutamic acid,
in an isolated biological sample from said individual.
